(19) Europäisches Patentamt
European Patent Office
Office européen des brevets

(11) EP 4 785 962 A1

(12) EUROPEAN PATENT APPLICATION
published in accordance with Art. 153(4) EPC

(43) Date of publication:
**05.08.2026 Bulletin 2026/32**

(21) Application number: **24870983.4**

(22) Date of filing: **27.09.2024**

(51) International Patent Classification (IPC):
***A61K 47/68*** (2017.01) ***C07K 16/30*** (2006.01)
***C07K 16/28*** (2006.01) ***A61P 35/00*** (2006.01)

(52) Cooperative Patent Classification (CPC):
**A61K 47/68; A61P 35/00; C07K 16/28; C07K 16/30**

(86) International application number:
**PCT/CN2024/121813**

(87) International publication number:
**WO 2025/067446 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.09.2023 CN 202311277590**
**26.09.2024 CN 202411346354**

(71) Applicant: **Fortvita Biologics Inc.**
**1205 Grand Cayman (KY)**

(72) Inventors:
- **XIONG, Huizhong**
**Suzhou, Jiangsu 215123 (CN)**
- **ZHANG, Xiguang**
**Suzhou, Jiangsu 215123 (CN)**
- **ZHU, Chenchen**
**Suzhou, Jiangsu 215123 (CN)**

(74) Representative: **Sagittarius IP**
**Marlow International**
**Parkway**
**Marlow SL7 1YL (GB)**

Remarks:
The complete document including Reference Table(s) and the Sequence Listing(s) can be downloaded from the EPO website

# (54) ANTIBODY-DRUG CONJUGATE TARGETING TROP2, AND PREPARATION METHOD AND USE THEREFOR

(57) The present disclosure is intended to provide a TROP2-targeted antibody-drug conjugate, a preparation method therefor, and use thereof. The TROP2-targeted antibody-drug conjugate according to the present disclosure shows strong tumor-specific cytotoxicity, and shows a stronger "bystander effect" in an in vivo experiment, and thus can be used as a medical drug, particularly a therapeutic single drug or combination drug against a tumor, a neoadjuvant therapy for a tumor, and the like.

EP 4 785 962 A1

## Description

### FIELD OF THE INVENTION

The present disclosure relates to an antibody-drug conjugate (ADC), and in particular to an antibody-drug conjugate targeting TROP2, a preparation method therefor, and use thereof.

### Background of the Invention

An ADC (antibody-drug conjugate) generally consists of three parts: an antibody, a payload (e.g., a bioactive small molecule), and a linker. The payload is covalently conjugated to the antibody via a linker. The antibody (e.g., monoclonal antibody) can specifically recognize a specific target on the surface of tumor cells, thereby guiding the ADC to the surface of cancer cells and allowing the ADC to enter the cancer cells through endocytosis. The payload is then released in the tumor microenvironment, thereby specifically killing the cancer cells without damaging normal tissues.

TROP2, a trophoblast cell-surface antigen, also known as tumor-associated calcium signal transducer (TACSTD2), is overexpressed in a variety of human epithelial cancers, including breast cancer, lung cancer, gastric cancer, colorectal cancer, pancreatic cancer, prostate cancer, cervical cancer, head and neck cancer, ovarian cancer, and the like (YeZhe Cheng et al., Frontiers in Oncology, Dec. 23, 2022; 12:951589).

Preclinical and clinical studies have demonstrated the use of anti-TROP2 antibody-drug conjugates (ADCs) (e.g., Trodelvy, an anti-human TROP2 antibody-SN-38 conjugate) for the treatment of cancer. Clinical results show that Trodelvy has achieved good therapeutic effects in the treatment of refractory solid tumors. The objective response rate (ORR) of Trodelvy reached 33% in patients with drug-resistant triple-negative breast cancer (TNBC). One of the important mechanisms of action of Trodelvy, SN-38 (payload) is linked by a pH-sensitive linker, which can be cleaved and specifically release SN-38 in the acidic tumor microenvironment. However, because the linker of Trodelvy is not stable enough, the maleimide-mediated linker is cleaved by thiol exchange under physiological conditions, so that the half-life of Trodelvy in serum is relatively short (about 1 day). Thus, Trodelvy may have relatively high off-target effects. In the art, there is still a need for antibody-drug conjugates targeting TROP2 for treatment.

### SUMMARY OF THE INVENTION

Therefore, one aspect of the present disclosure is to provide an antibody-drug conjugate having the following formula (I), or a stereoisomer, a pharmaceutically acceptable salt or a solvate thereof:

$$A(Z'-E-Q'-L_1-D)_p \quad \textbf{formula (I)},$$

wherein D is shown as formula (II) below:

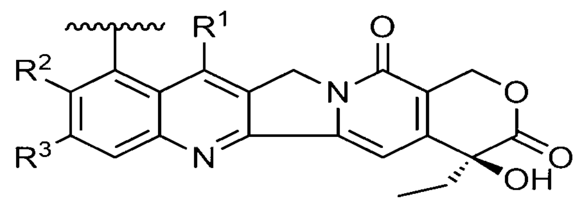

**Formula (II)**

wherein, $R^1$ is selected from H, $C_1$ -$C_6$ alkyl, $C_2$ -$C_6$ alkenyl, $C_2$ -$C_6$ alkynyl, $C_1$ -$C_6$ haloalkyl, $C_2$ -$C_6$ haloalkenyl and $C_2$ -$C_6$ haloalkynyl;

$R^2$ is selected from H, halogen, $C_1$ -$C_6$ alkyl, $C_1$ -$C_6$ haloalkyl, -$OR^6$ and -$SR^6$; $R^3$ is selected from H, halogen, CN, $C_1$ -$C_6$ alkyl, $C_1$ -$C_6$ haloalkyl and -$OR^6$; or $R^2$ and $R^3$ together form - $O(CH_2)_nO$- or -$O(CF_2)_nO$-, wherein n is 1 or 2;

$R^6$ is selected from H or $C_1$ -$C_4$ alkyl; and

$L_1$ is -$L_{1b}$-$L_{1a}$ -, wherein $L_{1b}$ is linked to Q', and $L_{1a}$ is linked to D,

wherein $L_{1a}$ is absent or -($C_1$ -$C_{10}$ alkylene)-;

$L_{1b}$ is absent, *-($C_1$ -$C_{10}$ alkylene)-C(O)N($R^5$)- or *-($C_1$ -$C_{10}$ alkylene)-N($R^5$)C(O)-;

wherein * indicates that the end is covalently linked to Q'; and $R^5$ is H or $C_1$ -$C_6$ alkyl,

Q' is -O- or -S-;

E is $-CH_2-NH-M-$, wherein the M is a peptide residue comprising 2 to 10 amino acids; wherein the peptide residue is optionally substituted with one or more (e.g., 2, 3, or 4) groups independently selected from $C_{1-6}$ alkyl and a polyol group; and wherein the N-terminus of the M is covalently attached to Z';

Z' is -C(=O)-$L_2$-Y'-, wherein Z' is covalently linked to E through the -C(=O) moiety shown and linked to A through the Y' moiety;

$L_2$ is selected from $-(CH_2)_m-(O-CH_2CH_2)_{m1}-(CH_2)_{m2}-$, $-(CH_2)_m-(O-CH_2CH_2)_{m1}-NHC(O)-(CH_2)_m-$ and $-(CH_2)_m-(O-CH_2CH_2)_{m1}-C(O)NH-(CH_2)_m-$, wherein m1 and m2 are independently selected from integers of 0 to 20, e.g., 0, 1, 2, 3, 4, 5, 6, 7, 8, 9, 10, 11, 12, 13, 14, 15 or 16; m is selected from integers of 1 to 10, e.g., 1, 2, 3, 4, 5, 6, 7 or 8, wherein the left end of these groups is linked to -C(=O)-, and the right end of these groups is linked to Y';

Y' is a group formed by

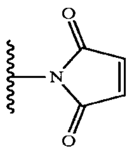

;

A represents an antibody or an antibody fragment, preferably an antigen-binding fragment, targeting TROP2; and

p is an average value of the drug-to-antibody ratio (i.e., average DAR) and is a value between 1 and 15, e.g., a value between 2 and 10, 2 and 8, 2 and 6, 2 and 5, 3 and 5, or 3.5 and 4.5.

In one embodiment, the present disclosure provides a TROP2-targeted antibody-drug conjugate having a structure of formula (I):

$$A(-Z'-E-Q'-L_1-D)_p$$ **formula (I),**

wherein D is shown as formula (II) below:

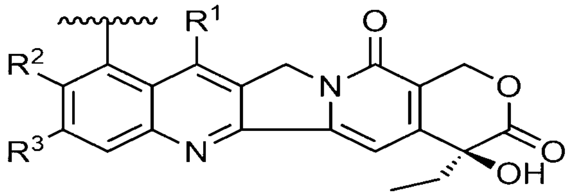

**Formula (II)**

wherein, $R^1$ is selected from -H or $C_1$-$C_4$ alkyl; $R^2$ is selected from -H, -F, $C_1$-$C_3$ alkyl or $C_1$-$C_3$ haloalkyl; $R^3$ is selected from -H, -F, -CN, $-OCH_3$, $-CH_3$ or $-CF_3$;

$L_1$ is independently unsubstituted or halogenated -($C_1$-$C_{10}$ alkylene)-;

Q' is -O- or -S-;

E is $-CH_2-NH-M$, the M being a peptide comprising 2 to 10 amino acids; wherein optionally the amino acids are substituted with one or more polyols; and wherein the N-terminus of the M is covalently attached to Z';

Z' is -C(=O)-$L_2$-Y';

$L_2$ is -($C_1$-$C_{10}$ alkylene)-;

Y' is formed from

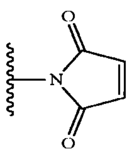

;

A represents an antibody or antibody fragment targeting TROP2; and

p is the average value of the drug-to-antibody ratio, with a value between 1-15,

the antibody or antibody fragment targeting TROP2 comprises a heavy chain and a light chain, wherein the heavy chain comprises a CDR1 having at least 80% sequence identity to the sequence set forth in the amino acid sequence

SEQ ID NO: 1, a CDR2 having at least 80% sequence identity to the sequence set forth in the amino acid sequence SEQ ID NO: 2, and a CDR3 having at least 80% sequence identity to the sequence set forth in the amino acid sequence SEQ ID NO: 3;

the light chain comprises a CDR1 having at least 80% sequence identity to the sequence set forth in the amino acid sequence SEQ ID NO: 4, a CDR2 having at least 80% sequence identity to the sequence set forth in the amino acid sequence SEQ ID NO: 5, and a CDR3 having at least 80% sequence identity to the sequence set forth in the amino acid sequence SEQ ID NO: 6.

In another aspect, the present disclosure provides a composition comprising one or more antibody-drug conjugates according to the present disclosure.

In yet another aspect, the present disclosure provides use of the antibody-drug conjugate according to the present disclosure or the composition according to the present disclosure in manufacture of a medicament for treating a cell proliferative disease or disorder or inhibiting abnormal cell growth.

In yet another aspect, the present disclosure provides a method for preparing the antibody-drug conjugate according to the present disclosure, which comprises:

(i) preparing a linker-payload conjugate having a structure of the following formula (III):

$$Z\text{-}E\text{-}Q'\text{-}L_1\text{-}D \text{ formula} \qquad (III)$$

wherein the symbols are as defined herein; and

(ii) reacting the linker-payload conjugate obtained in step (i) with the TROP2-targeted antibody or antibody fragment.

## BRIEF DESCRIPTION OF THE DRAWINGS

**FIG. 1** shows a schematic structural diagram of a TROP2-targeted ADC.

**FIGs. 2A and 2B** show the detection of cytotoxic effect of TROP2-targeted ADCs on human hypopharyngeal carcinoma squamous cell line FaDu in vitro.

**FIGs. 3A and 3B** show the detection of the cytotoxic effect of TROP2-targeted ADCs on human pancreatic cancer cell line BxPC-3 in vitro.

**FIG. 4** shows the test results of the in vitro bystander effect of TROP2-targeted ADCs.

**FIGs. 5A and 5B** show the anti-tumor effect of TROP2-targeted ADCs in an in vivo mouse xenograft tumor model of human gastric cancer cell line NCI-N87.

**FIG. 6** shows the anti-tumor effect of TROP2-targeted ADCs in an in vivo mouse xenograft tumor model of a human lung cancer cell line HCC827, wherein A is a low-dose group and B is a high-dose group.

**FIG. 7** shows the anti-tumor effect of TROP2-targeted ADCs in an in vivo mouse xenograft tumor model of human lung cancer cell line LK2.

**FIG. 8** shows the anti-tumor effect of TROP2-targeted ADCs in an in vivo mouse xenograft tumor model of human pancreatic cancer cell line BxPC-3.

**FIG. 9** shows the anti-tumor effect of a TROP2-targeted ADC in an in vivo human mouse xenograft tumor model of lung cancer cell line EBC-1.

**FIG. 10A** shows a schematic of detection method for the stability of a TROP2-targeted ADC in mice.

FIG. 10 B shows the detection results of the stability of TROP2-targeted ADCs in mice.

## DETAILED DESCRIPTION OF THE INVENTION

Various embodiments of the TROP2-targeted ADC of the present disclosure and methods for using the same will be described below with reference to the accompanying drawings. The following embodiments are given as exemplary embodiments of the embodiments of the present disclosure and are not intended to limit the scope of the present disclosure.

In one aspect, the present disclosure provides a TROP2-targeted antibody-drug conjugate (also referred to as "anti-TROP2 antibody-drug conjugate" or "TROP2-targeted ADC"). In the present disclosure, the TROP2-targeted antibody-drug conjugate comprises a TROP2-targeted antibody and a drug linked by a linker.

Anti-TROP2 antibody-drug conjugate

In the present disclosure, the present disclosure provides a TROP2-targeted antibody-drug conjugate, wherein the TROP2-targeted ADC is linked to the TROP2-targeted antibody and the camptothecin derivative by a peptide linker.

According to the present disclosure, the antibody-drug conjugate targeting TROP2 and the existing Ds-1062(hTINA1-DXd) (datopotamab deruxtecan, a novel TROP2-directed antibody-drug conjugate, demonstrates potent antitumor activity by efficient drug delivery to tumor cells, Daisuke Okajima et al., Mol Cancer Ther. 2021 Dec;20(12):2329-2340), the TROP2-targeted ADC of the present disclosure exhibited a stronger tumor inhibitory effect in a plurality of in vivo xenograft tumor models. In in vitro experiments, it was found that the molecules of the present disclosure have a better "bystander effect", which may be because the ADC drugs of the present disclosure have better hydrophobic properties and can pass through the cell membrane more effectively. In terms of toxicity, it was observed that the TROP2-targeted ADC of the present disclosure did not cause severe weight loss in mice, and showed good tolerability overall, which was not weaker than that of Ds-1062.

Compared with the existing Trodelvy (Trop-2 is a novel target for solid cancer therapy with sacituzumab govitecan (IMMU-132)), an antibody-drug conjugate (ADC), David M Goldenberg et al., Oncotarget. 2015 Sep 8;6(26):22496-512), the TROP2-targeted ADC of the present disclosure has better stability (see the literature). The TROP2-targeted ADC of the present disclosure has a stronger tumor inhibitory effect.

In addition, the small molecule drug of the present disclosure has stronger hydrophobic properties, and can better penetrate the cell membrane and enter cells, thereby acting on topoisomerase in the cells and inhibiting cell growth. The TROP2-targeted ADC according to the present disclosure shows a stronger "bystander effect" in an in vivo experiment, thereby having a stronger tumor killing effect. Based on the above points, it can be inferred that the TROP2-targeted ADC according to the present disclosure will show a better tumor inhibitory effect clinically.

In the present disclosure, the TROP2-targeted ADC shows strong tumor-specific cytotoxicity, and thus can be used as a medical drug, particularly a single drug or a combination drug for tumor treatment, a neoadjuvant therapy for tumors, and the like.

In one aspect, the present disclosure provides an antibody-drug conjugate having the following formula (I), or a stereoisomer, a pharmaceutically acceptable salt, or a solvate thereof:

$$A\left(-Z'-E-Q'-L_1-D\right)_p \quad \textbf{formula (I)},$$

wherein D is shown as formula (II) below:

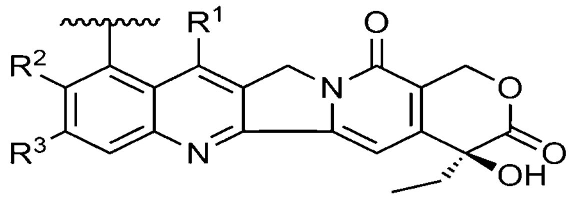

**Formula (II)**

wherein, $R^1$ is selected from H, $C_1$ -$C_6$ alkyl, $C_2$ -$C_6$ alkenyl, $C_2$ -$C_6$ alkynyl, $C_1$ -$C_6$ haloalkyl, $C_2$ -$C_6$ haloalkenyl and $C_2$ -$C_6$ haloalkynyl;

$R^2$ is selected from H, halogen, $C_1$ -$C_6$ alkyl, $C_1$ -$C_6$ haloalkyl, -$OR^6$ and -$SR^6$; $R^3$ is selected from H, halogen, CN, $C_1$ -$C_6$ alkyl, $C_1$ -$C_6$ haloalkyl and -$OR^6$; or $R^2$ and $R^3$ together form - $O(CH_2)_nO$- or -$O(CF_2)_nO$-, wherein n is 1 or 2;

$R^6$ is selected from H or $C_1$ -$C_4$ alkyl; and

$L_1$ is -$L_{1b}$-$L_{1a}$-, wherein $L_{1b}$ is linked to Q', and $L_{1a}$ is linked to D;

wherein $L_{1a}$ is absent or -($C_1$ -$C_{10}$ alkylene)-;

$L_{1b}$ is absent, *-($C_1$ -$C_{10}$ alkylene)-C(O)N($R^5$)- or *-($C_1$ -$C_{10}$ alkylene)-N($R^5$)C(O)-; wherein * indicates that the end is covalently linked to Q'; and $R^5$ is H or $C_1$ -$C_6$ alkyl,

Q' is -O- or -S-;

E is -$CH_2$-NH-M-, wherein the M is a peptide residue comprising 2 to 10 amino acids; wherein the peptide residue is optionally substituted with one or more (e.g., 2, 3, or 4) groups independently selected from $C_{1-6}$ alkyl and a polyol group; and wherein the N-terminus of the M is covalently attached to Z';

Z' is -C(=O)-$L_2$-Y'-, wherein Z' is covalently linked to E through the -C(=O) moiety shown and linked to A through the Y' moiety;

$L_2$ is selected from $-(CH_2)_m-(O-CH_2CH_2)_{m1}-(CH_2)_{m2}-$, $-(CH_2)_m-(O-CH_2CH_2)_{m1}-NHC(O)-(CH_2)_m-$ and $-(CH_2)_m-(O-CH_2CH_2)_{m1}-C(O)NH-(CH_2)_m-$, wherein m1 and m2 are independently selected from an integer of 0 to 20, e.g., 0, 1, 2, 3, 4, 5, 6, 7, 8, 9, 10, 11, 12, 13, 14, 15 or 16; m is selected from integers of 1 to 10, e.g., 1, 2, 3, 4, 5, 6, 7 or 8, wherein the left end of these groups is linked to -C(=O)-, and the right end of these groups is linked to Y';

Y' is a group formed by

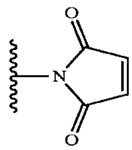
;

A represents an antibody or an antibody fragment, preferably an antigen-binding fragment, targeting TROP2; and p is an average value of the drug-to-antibody ratio (i.e., average DAR) and is a value between 1 and 15, e.g., a value between 2 and 10, 2 and 8, 2 and 6, 2 and 5, 3 and 5, or 3.5 and 4.5.

It will be appreciated that the N-terminus of M in the group E ($-CH_2-NH-M-$) as described above is covalently attached to Z', such that "$-CH_2-$" shown in E is covalently linked to Q'.

It should be understood that Y' is a group formed by

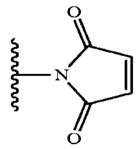
,

which means that it is a group formed by the reaction of

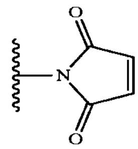

with A (an antibody or a fragment thereof), which is covalently linked to A.

In some embodiments, the M is a peptide residue of 2 to 10 amino acids, i.e., a peptide residue consisting of 2 to 10 amino acids; which is optionally substituted as described above.

In an embodiment, the present disclosure provides a TROP2-targeted antibody-drug conjugate of formula (I), or a stereoisomer, a pharmaceutically acceptable salt or a solvate thereof:

$$A(-Z'-E-Q'-L_1-D)_p$$ **formula (I),**

wherein D is shown as formula (II) below:

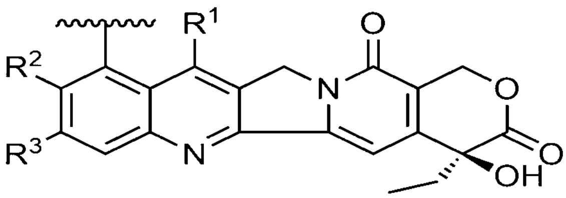
**Formula (II)**

wherein, $R^1$ is selected from -H or $C_1$-$C_4$ alkyl; $R^2$ is selected from -H, -F, $C_1$-$C_3$ alkyl or $C_1$-$C_3$ haloalkyl; $R^3$ is selected from -H, -F, -CN, $-OCH_3$, $-CH_3$ or $-CF_3$;

$L_1$ is independently unsubstituted or halogenated -($C_1$-$C_{10}$ alkylene)-;

Q' is -O- or -S-;

E is $-CH_2-NH-M$, the M being a peptide comprising 2 to 10 amino acids; wherein optionally the amino acids are

substituted with one or more polyols; and wherein the N-terminus of the M is covalently attached to Z';

Z' is -C(=O)-$L_2$-Y';

$L_2$ is -($C_1$ -$C_{10}$ alkylene)-;

Y' is formed from

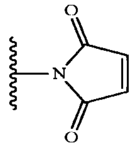

;

A represents an antibody or antibody fragment targeting TROP2; and

p is the average value of the drug-to-antibody ratio and has a value between 1 and 15.

In some embodiments, the present disclosure provides the TROP2-targeted antibody-drug conjugate of formula (I) as defined above.

In some embodiments, Y' is

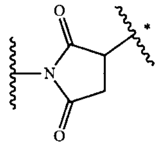

,

wherein * represents a site covalently linked to A.

In some embodiments, $L_2$ is -$(CH_2)_m$ -, wherein m is an integer selected from the group consisting of integers of 1-10, for example, 1, 2, 3, 4, 5, 6, 7, or 8, and is preferably 5.

In an embodiment according to the present disclosure, Z' is formed by:

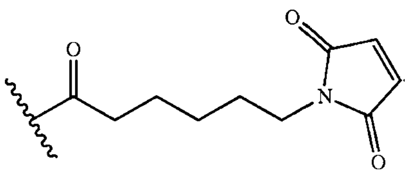

or

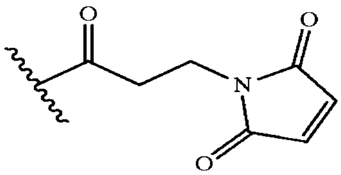

.

In an embodiment, Z' is:

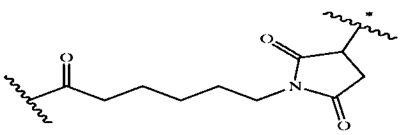

or

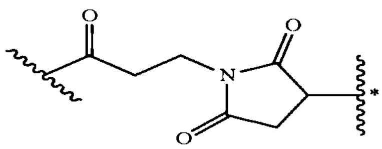

,

wherein * represents a site for covalent linking to A.

In some embodiments, M is a peptide comprising 2, 3, or 4 amino acids, each amino acid of M is an L amino acid, or at least one amino acid of M is a D amino acid.

In some embodiments, M is a peptide having 2, 3, or 4 amino acids, i.e., a peptide consisting of 2, 3, or 4 amino acids.

It will be understood that reference to peptides and amino acid residues as part of a structure refers to peptide residues or amino acid residues, as is well known to those skilled in the art.

In some embodiments, the amino acid is selected from, for example, glycine, alanine, valine, glutamine, glutamic acid, phenylalanine, leucine, tyrosine, lysine, citrulline, serine, tryptophan, aspartic acid, asparagine, isoleucine, arginine, and proline, and the glutamine or glutamic acid is optionally substituted with a polyol group (e.g., one polyol group) and optionally substituted with $C_{1-6}$ alkyl (e.g., one $C_{1-6}$ alkyl).

In some embodiments, M comprises one or more amino acids selected from glycine, alanine, valine, glutamine, glutamic acid, phenylalanine, and leucine, and the glutamine or glutamic acid is optionally substituted with a polyol. In some embodiments, M comprises amino acids selected from glycine, alanine, valine, glutamine, glutamic acid, phenylalanine, and leucine, and the glutamine or glutamic acid is optionally substituted with a polyol.

In some embodiments, the amino acid is selected from glycine, alanine, valine, glutamine, glutamic acid, phenylalanine, and leucine, and the glutamine or glutamic acid is optionally substituted with one polyol group and optionally substituted with one $C_{1-6}$ alkyl.

In some embodiments, the substituted glutamine or glutamic acid has the structure shown below:

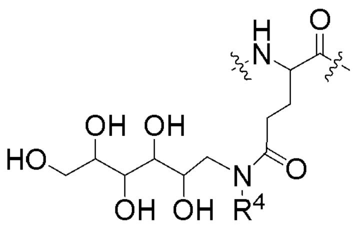 (G-1a), 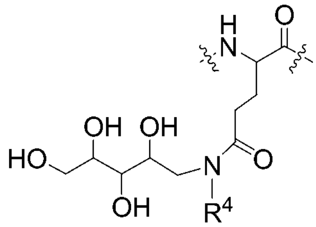 (G-1b),

wherein $R^4$ is H or $C_1$ -$C_6$ alkyl;
preferably

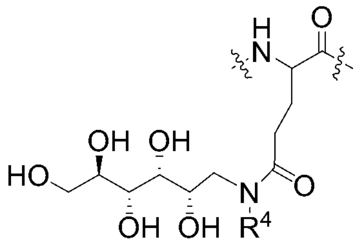 (G-1c)

wherein $R^4$ is H or $C_1$ -$C_6$ alkyl.

In some embodiments, the substituted glutamine or glutamic acid has the structure shown below:

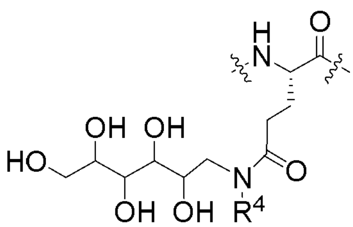 (G-2a), 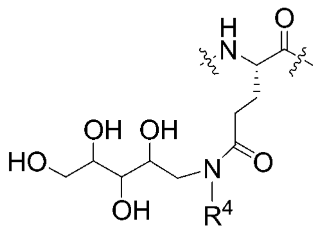 (G-2b),

wherein $R^4$ is H or $C_1$ -$C_6$ alkyl;
preferably

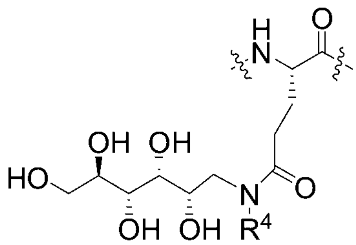 (G-2c)

wherein $R^4$ is H or $C_1$ -$C_6$ alkyl.

In some embodiments, M comprises an amino acid having the following structure:

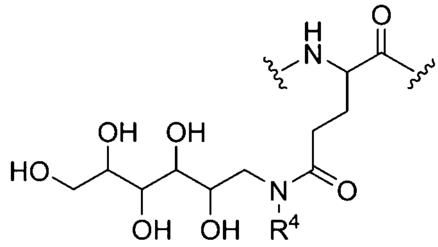

,

wherein $R^4$ is -H or $C_1$ -$C_6$ alkyl.

In some embodiments, M comprises an amino acid having the following structure:

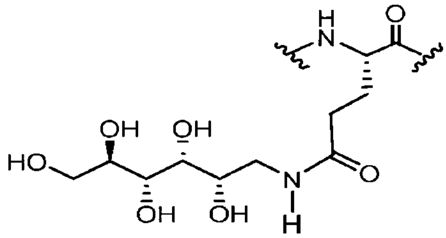

.

In some embodiments, M comprises or is a peptide residue selected from: -Ala-Val-*, -Val-Ala-*, -Gly-Gly-*, -Leu-Ala-*, -Ala-Leu-*, -Ala-Ala-*, -Phe-Gln-*, -Gln-Phe-*, -Leu-Gln-*, -Gln-Leu-*, -Val-Gln-*, -Phe-Ala-*, -Ala-Phe-*, -Gln-Val-*, -Ala-Ala-Ala-*, -Gly-Gly-Gly-*, -Ala-Val-Ala-*, -Gly-Val-Gly-*, -Ala-Val-Gly-*, -Gly-Phe-Gly-*, -Lys-Phe-Gly-*, -Leu-Ala-Leu-*, -Val-Ala-Leu-*, -Leu-Ala-Val-*, -Val-Ala-Val-*, -Ala-Val-Gln-*, -Ala-Val-Ala-Gly-*, -Gly-Phe-Gly-Gly-*, -Gly-Gly-Phe-Gly-*, -Gly-Phe-Gly-Gln-*, -Ala-Val-Gly-Gly-*, -Ala-Ala-Ala-Ala-*, -Ala-Val-Ala-Ala-*, -Ala-Leu-Ala-Leu-*, -Leu-Ala-Leu-Ala-*, -Gly-Phe-Leu-Gly-*, -Gly-Phe-Gly-Gln-*, and -Gly-Leu-Phe-Gly-*, wherein * indicates the N-terminus of the peptide covalently attached to Z'.

In some embodiments, Gln is optionally substituted with one polyol group and optionally substituted with one $C_{1-6}$ alkyl.

In some embodiments, Gln is unsubstituted.

In some embodiments, Gln is substituted with one polyol group and/or substituted with one $C_{1-6}$ alkyl group. In some embodiments, M is selected from -L-Ala-D-Val-*, -L-Val-D-Ala-*, -L-Ala-L-Val-*, -L-Ala-D-Ala-*, -L-Ala-D-Ala-L-Ala-*, -L-Ala-L-Ala-L-Ala-*, -L-Ala-D-Val-L-Ala-*, - L-Ala-D-Ala-Gly-*, -L-Ala-D-Val-Gly-*, -L-Ala-L-Val-Gly-Gly-*, -L-Ala-L-Val-L-Gln-*, or - Gly-L-Phe-Gly-L-Gln-*, wherein * indicates the N-terminus of the peptide covalently attached to Z'.

In some embodiments, -E- has one of the following structures, wherein * indicates the N-terminus of the peptide covalently attached to Z':

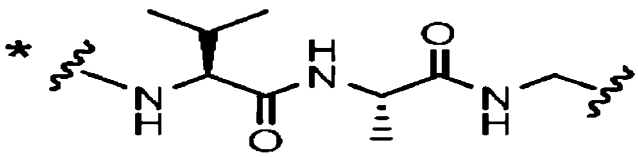

,

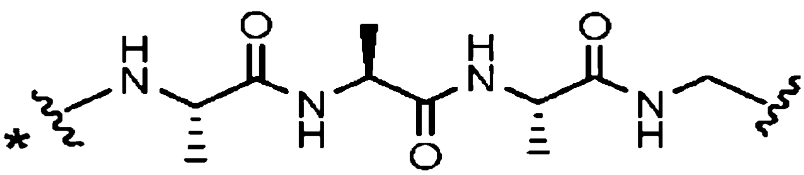

,

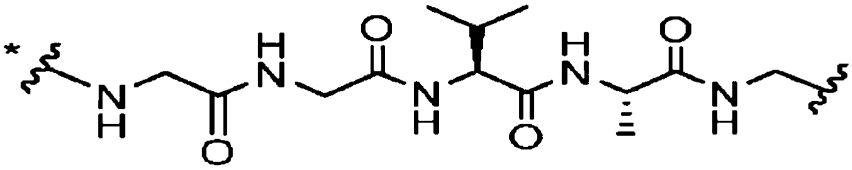

,

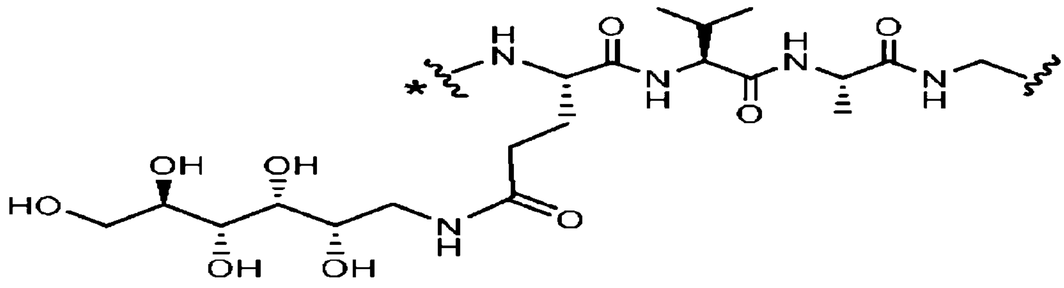

,

or

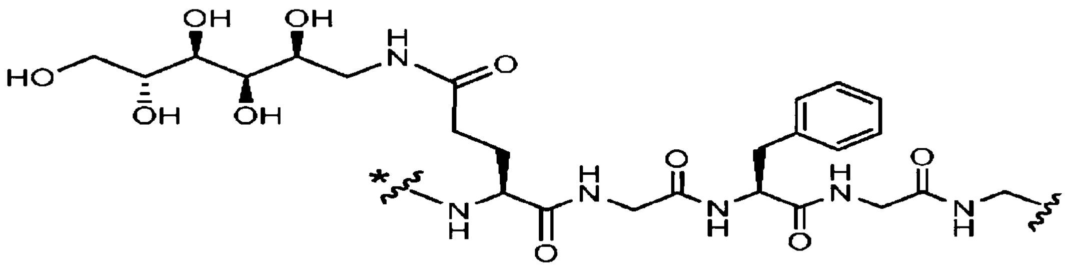

.

In some embodiments, Z'-E is formed from one of the following structures:

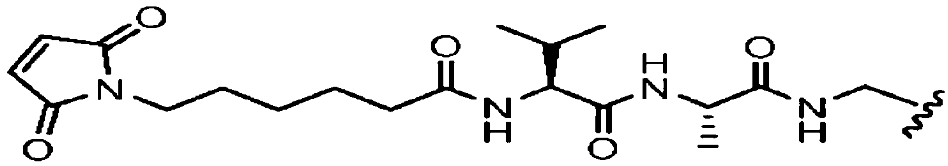

,

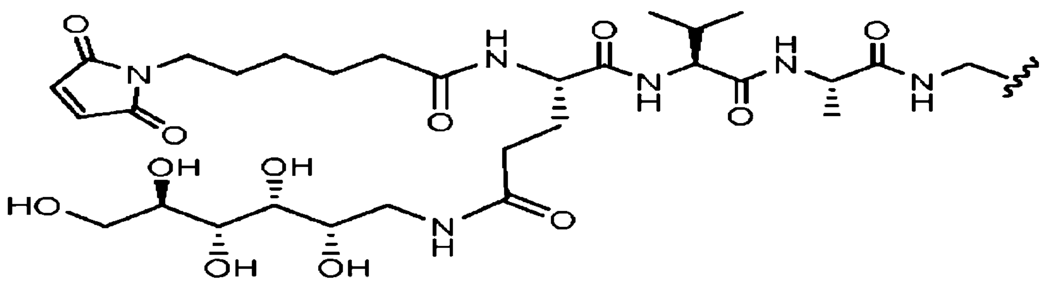

,

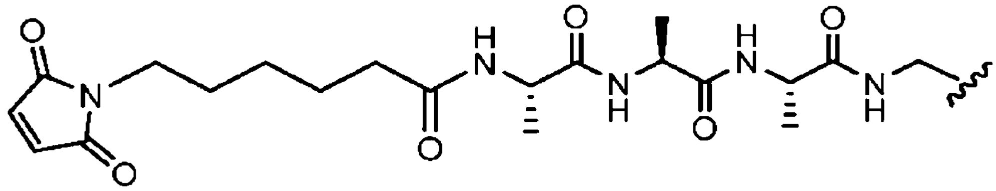

,

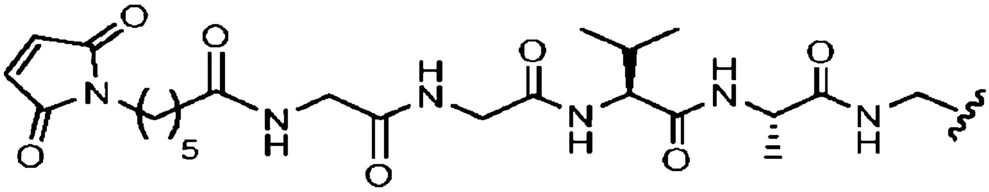

,

or

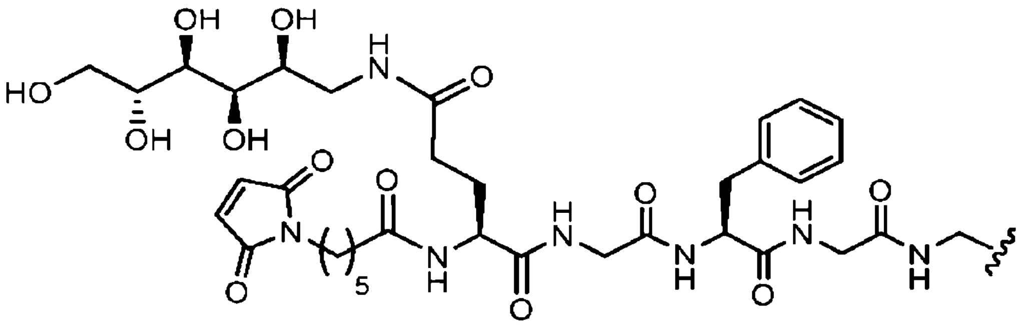

In some embodiments, Z'-E is selected from the following structures, wherein * represents a point of attachment to A:

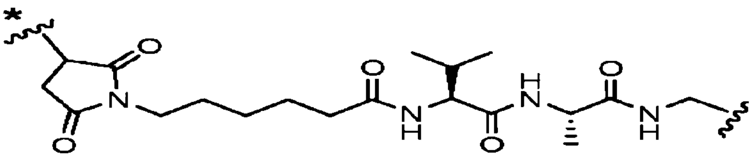

,

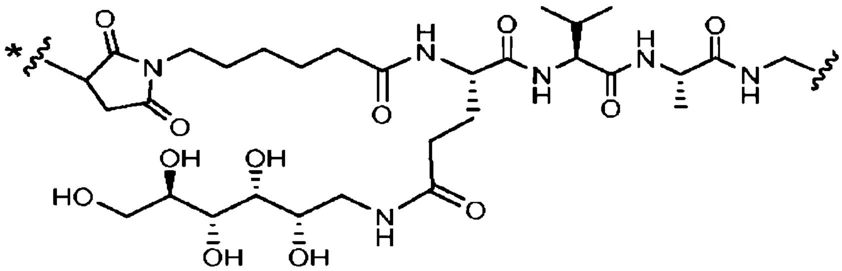

,

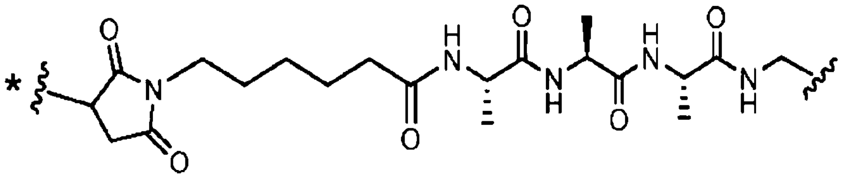

,

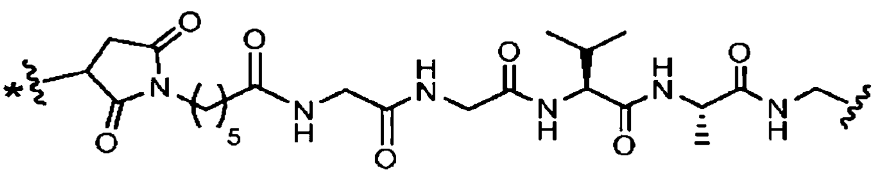

,

or

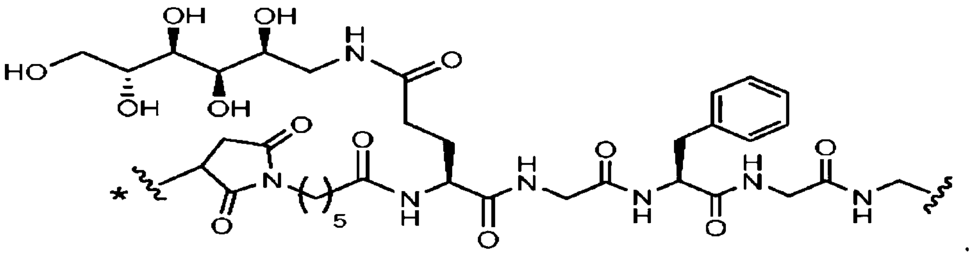

In some embodiments, at least one of $R^1$, $R^2$, and $R^3$ is not -H.

In some embodiments, $R^1$ is independently -H, methyl, ethyl, or propyl.

In some embodiments, $R^2$ is independently -H, -F, methyl or ethyl; and $R^3$ is independently-H or -F.

In some embodiments, D is represented by the following structure:

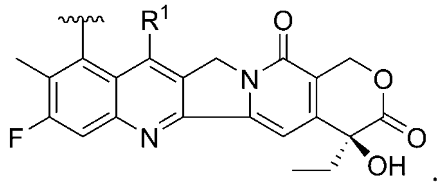

In some embodiments, D is represented by the following structure:

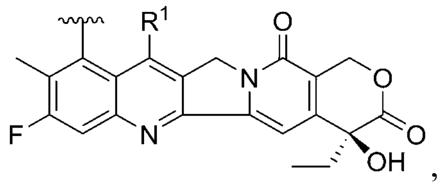

wherein $R^1$ is -H or $C_1$ -$C_3$ alkyl.

In some embodiments, D is represented by the following structure:

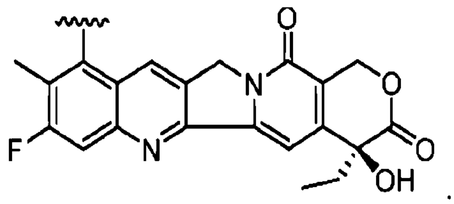

In some embodiments, $L_1$ is $-CH_2-$, $-CH_2CH_2-$, $-CH_2CH_2CH_2-$ or $-CH_2CH_2CH_2CH_2-$.

In some embodiments, $L_1$-Q' is $-CH_2CH_2CH_2CH_2O-$, $-CH_2CH_2CH_2O-$, $-CH_2CH_2O-$, - $CH_2CH_2CH_2CH_2S-$, $-CH_2CH_2CH_2S-$, or $-CH_2CH_2S-$.

In some embodiments, D-$L_1$ is represented by the following structure

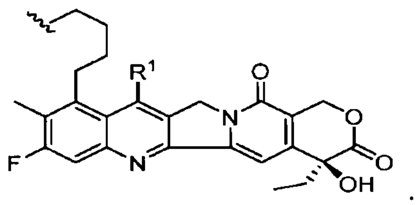

In some embodiments, D-$L_1$ is represented by the following structure

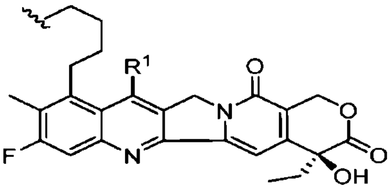,

and $R^1$ is -H or $C_1$ -$C_3$ alkyl.

In some embodiments, Q' is -O-.

In some embodiments, D-$L_1$ -Q'- has one of the following structures:

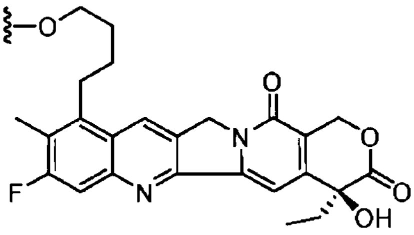 or 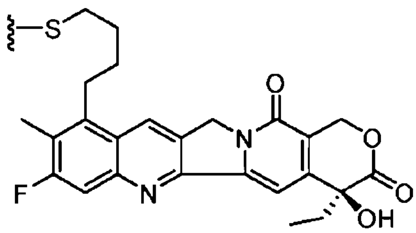.

In some embodiments, D-$L_1$ -Q'-E-Z'- is formed from one of the following structures:

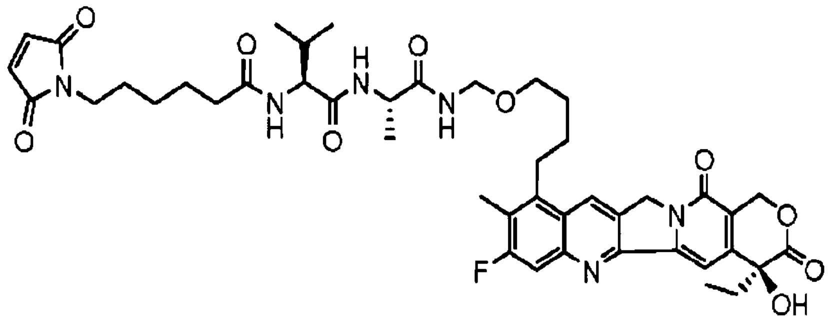,

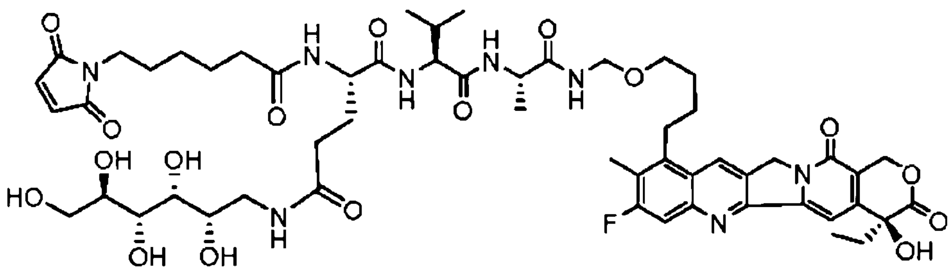,

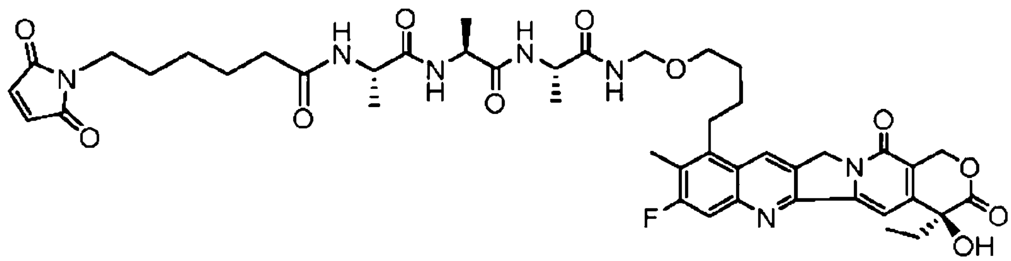,

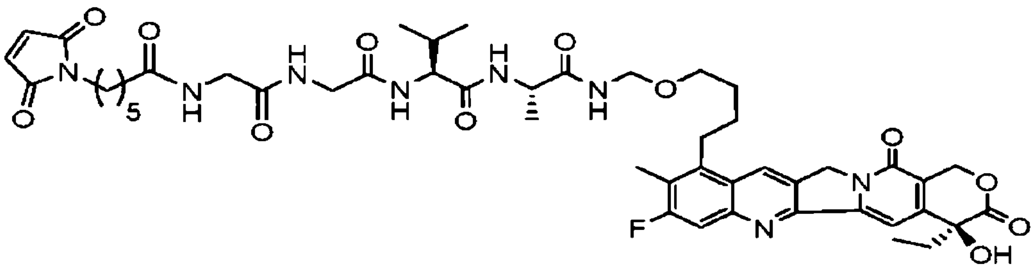

,

or

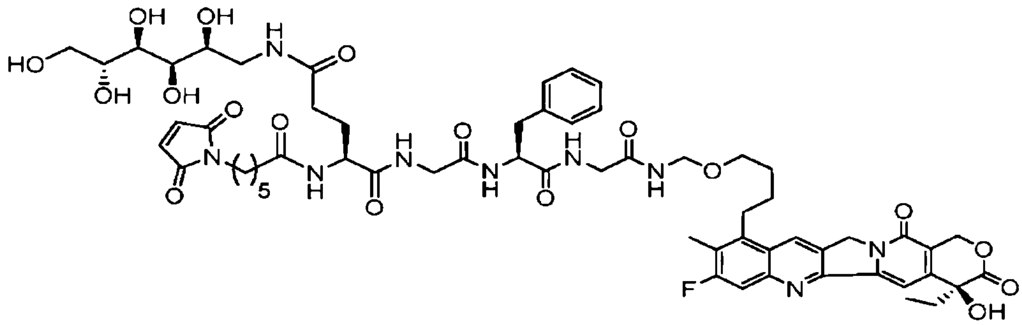

.

In some embodiments,

$$A\left(Z'-E-Q'-L_1-D\right)_p$$

is the following structure, wherein A is as defined herein, e.g., a monoclonal antibody, p is the average of drug-to-antibody ratios (DARs), and p is a value between 1-15, 1-10, 2-8, or 4-8 (inclusive):

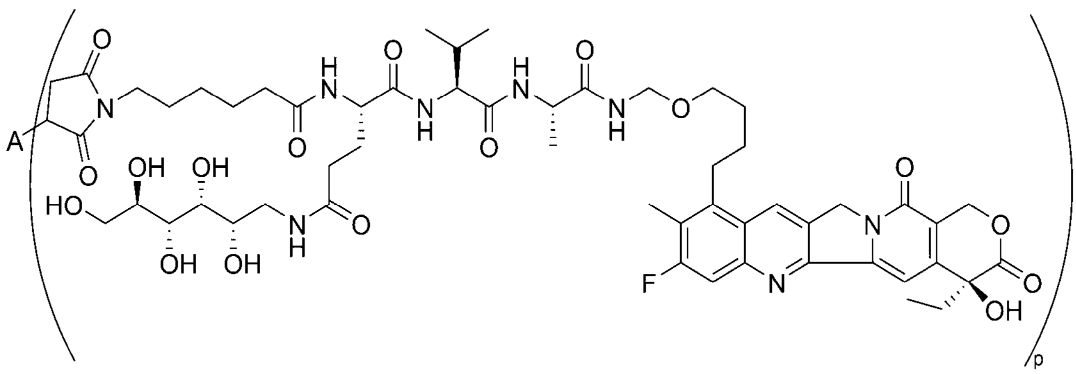

.

In some embodiments, the linker L is linked to the antibody (A) via a maleimide moiety by - S-, wherein the -S- is from a cysteine side chain of A.

In one aspect, the present invention provides a compound of formula (I'), or a stereoisomer, a pharmaceutically acceptable salt, or a solvate thereof:

$$A\left(Z'-E-Q'-L_1-D\right)_q \quad (I')$$

wherein q is DAR and is an integer selected from 1-15, e.g., 1, 2, 3, 4, 5, 6, 7, 8, 9, 10, 11, 12, 13, 14, or 15; and the other symbols, e.g., A, Z', E, Q', $L_1$ and D, are as defined herein for formula (I).

The TROP2-targeted antibody or antibody fragment of the present disclosure may be a mammalian-derived

(e.g., human or mouse), humanized, or chimeric TROP2-targeted antibody or antibody fragment. Preferably, the TROP2-targeted antibody or antibody fragment is a monoclonal antibody recombinantly produced from cells genetically modified according to techniques widely described in the prior art.

When A is an antibody targeting TROP2, it is preferably a human IgG, e.g., an IgG1, IgG2, IgG3 or IgG4 antibody.

The RS7 antigen has been named EGP-1 (epithelial glycoprotein-1) following the recommendations of the Third International Association for the Study of Lung Cancer (IASLC) Workshop on Tumor and Differentiation Antigens. At least one epitope related to EGP-1 is also referred to in the literature as TROP2. In a preferred embodiment, the antibody or antibody fragment of the present disclosure binds to the same epitope as the murine RS7 antibody disclosed by Stein et al. (Stein et al., Antibody Immunocon J. Radiopharm. 4: 703 (1991), which is incorporated by reference in its entirety) and other prior studies. Alternatively, the antibody or fragment may bind to a different epitope than the murine RS7 antibody disclosed by Stein.

In some embodiments, the TROP2-targeted antibody or antibody fragment according to the present disclosure comprises a heavy chain and a light chain, wherein the heavy chain comprises a CDR1 having at least 80% sequence identity (preferably at least 90% sequence identity, such as at least 95%, at least 96%, at least 97%, at least 98%, at least 99% or even 100% sequence identity) to the sequence set forth in the amino acid sequence SEQ ID NO: 1, a CDR2 having at least 80% sequence identity (preferably at least 90% sequence identity, such as at least 95%, at least 96%, at least 97%, at least 98%, at least 99% or even 100% sequence identity) to the sequence set forth in the amino acid sequence SEQ ID NO: 2, and a CDR3 having at least 80% sequence identity (preferably at least 90% sequence identity, such as at least 95%, at least 96%, at least 97%, at least 98%, at least 99% or even 100% sequence identity) to the sequence set forth in the amino acid sequence SEQ ID NO: 3;
the light chain comprises a CDR1 having at least 80% sequence identity (preferably at least 90% sequence identity, such as at least 95%, at least 96%, at least 97%, at least 98%, at least 99% or even 100% sequence identity) to the sequence set forth in the amino acid sequence SEQ ID NO: 4, a CDR2 having at least 80% sequence identity (preferably at least 90% sequence identity, such as at least 95%, at least 96%, at least 97%, at least 98%, at least 99% or even 100% sequence identity) to the sequence set forth in the amino acid sequence SEQ ID NO: 5, and a CDR3 having at least 80% sequence identity (preferably at least 90% sequence identity, such as at least 95%, at least 96%, at least 97%, at least 98%, at least 99% or even 100% sequence identity) to the sequence set forth in the amino acid sequence SEQ ID NO: 6.

For example, the antibody or fragment thereof targeting TROP2 comprises a heavy chain and a light chain, wherein the heavy chain comprises a CDR1 comprising the amino acid sequence GYTFTNYGM (SEQ ID NO: 1); a CDR2 comprising the amino acid sequence WINTYTGEPTYTDDFKG (SEQ ID NO: 2); and a CDR3 comprising the amino acid sequence GGFGSSYWYFDV (SEQ ID NO: 3), and the light chain comprises a CDR1 comprising the amino acid sequence KASQDVSIAVA (SEQ ID NO: 4); a CDR2 comprising the amino acid sequence SASYRYT (SEQ ID NO: 5); and a CDR3 comprising the amino acid sequence QQHYITPLT (SEQ ID NO: 6).

For example, a humanized antibody or fragment thereof targeting TROP2, wherein the complementarity determining regions (CDRs) of the light chain variable region of the humanized RS7 MAb comprise a CDR1 comprising the amino acid sequence KASQDVSIAVA; a CDR2 comprising the amino acid sequence SASYRYT; and a CDR3 comprising the amino acid sequence QQHYITPLT. In some embodiments of the humanized antibody or the fragment thereof targeting TROP2 of the present disclosure, the CDRs of the heavy chain variable region of the humanized RS7 MAb comprise a CDR1 comprising an amino acid sequence of GYTFTNYGM, a CDR2 comprising an amino acid sequence of WINTYTGEPTYTDDFKG, and a CDR3 comprising an amino acid sequence of GGFGSSYWYFDV. Also preferably, the humanized antibody or the fragment thereof further comprises FRs of light and heavy chain constant regions of a human antibody.

In a specific embodiment, the antibody or a fragment thereof targeting TROP2 comprises a variable domain of a light chain and a variable domain of a heavy chain, for example, the variable domain of the heavy chain comprises a CDR1 comprising an amino acid sequence of GYTFTNYGM (SEQ ID NO: 1), a CDR2 comprising an amino acid sequence of WINTYTGEPTYTDDFKG (SEQ ID NO: 2), and a CDR3 comprising an amino acid sequence of GGFGSSYWYFDV (SEQ ID NO: 3), and the variable domain of the light chain comprises a CDR1 comprising an amino acid sequence of KASQDVSIAVA (SEQ ID NO: 4), a CDR2 comprising an amino acid sequence of SASYRYT (SEQ ID NO: 5), and a CDR3 comprising an amino acid sequence of QQHYITPLT (SEQ ID NO: 6).

In a specific embodiment, the variable domain of the light chain of the TROP2-targeted antibody or fragment thereof has at least 80% sequence identity, preferably at least 90% sequence identity, such as at least 95%, at least 96%, at least 97%, at least 98%, at least 99% or even 100% sequence identity, with the amino acid sequence SEQ ID NO: 8; and the variable domain of the heavy chain of the TROP2-targeted antibody or fragment thereof has at least 80% sequence identity, preferably at least 90% sequence identity, such as at least 95%, at least 96%, at least 97%, at least 98%, at least 99% or even 100% sequence identity, with the amino acid sequence SEQ ID NO: 7.

In a specific embodiment, the antibody or the fragment thereof targeting TROP2 comprises a variable domain of a light chain and a variable domain of a heavy chain, wherein the variable domain of the light chain comprises or consists of the amino acid sequence set forth in SEQ ID NO: 8, and the variable domain of the heavy chain comprises or consists of the

amino acid sequence set forth in SEQ ID NO: 7.

In a specific embodiment, the TROP2-targeted antibody or fragment thereof comprises a heavy chain and a light chain, wherein the light chain of the TROP2-targeted antibody or fragment thereof has at least 80% sequence identity, preferably at least 90% sequence identity, such as at least 95%, at least 96%, at least 97%, at least 98%, at least 99% or even 100% sequence identity, with the amino acid sequence SEQ ID NO: 10; and the heavy chain of the TROP2-targeted antibody or fragment thereof has at least 80% sequence identity, preferably at least 90% sequence identity, such as at least 95%, at least 96%, at least 97%, at least 98%, at least 99% or even 100% sequence identity, with the amino acid sequence SEQ ID NO: 9.

In a specific embodiment, the antibody or the fragment thereof targeting TROP2 comprises a heavy chain and a light chain, wherein the heavy chain comprises or consists of the amino acid sequence set forth in SEQ ID NO: 9, and the light chain comprises or consists of the amino acid sequence set forth in SEQ ID NO: 10. In a specific embodiment, A is the hRS7 antibody as shown in the examples.

In a specific embodiment, the hRS7 antibody is a humanized antibody targeting TROP2 in IgG1 form, which has a light chain, the sequence of which is SEQ ID NO: 10 and a heavy chain, the sequence of which is SEQ ID NO: 9.

In a specific embodiment, the antibody targeting TROP2 is a full-length antibody. In a specific embodiment, the antibody or the fragment thereof targeting TROP2 comprises or consists of two heavy chains and two light chains.

In a specific embodiment, the antibody targeting TROP2 is a multispecific antibody, e.g., a bispecific antibody.

In a specific embodiment, the antibody targeting TROP2 is a monoclonal antibody.

In a specific embodiment, the antibody targeting TROP2 is a chimeric antibody or a humanized antibody.

The related sequences of the hRS7 antibody are shown as an example in Table 1 below.

Table 1

| Antibody name hRS7-IgG1 | | |
|---|---|---|
| ITEM | Sequence (5' to 3') | SEQ ID NO: |
| HCDR1 | GYTFTNYGM | 1 |
| HCDR2 | WINTYTGEPTYTDDFKG | 2 |
| HCDR3 | GGFGSSYWYFDV | 3 |
| VH | QVQLQQSGSELKKPGASVKVSCKASGYTFTNYGMNWVKQAPGQGLKWMGWINTYTGEPTYTDDFKGRFAFSLDTSVSTAYLQISSLKADDTAVYFCARGGFGSSYWYFDVWGQGSLVTVSS | 7 |
| HC | QVQLQQSGSELKKPGASVKVSCKASGYTFTNYGMNWVKQAPGQGLKWMGWINTYTGEPTYTDDFKGRFAFSLDTSVSTAYLQISSLKADDTAVYFCARGGFGSSYWYFDVWGQGSLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKRVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK | 9 |
| LCDR1 | KASQDVSIAVA | 4 |
| LCDR2 | SASYRYT | 5 |
| LCDR3 | QQHYITPLT | 6 |
| VL | DIQLTQSPSSLSASVGDRVSITCKASQDVSIAVAWYQQKPGKAPKLLIYSASYRYTGVPDRFSGSGSGTDFTLTISSLQPEDFAVYYCQQHYITPLTFGAGTKVEIK | 8 |

(continued)

| Antibody name hRS7-IgG1 | | |
|---|---|---|
| ITEM | Sequence (5' to 3') | SEQ ID NO: |
| LC | DIQLTQSPSSLSASVGDRVSITCKASQDVSIAVAWYQQKPG KAPKLLIYSASYRYTGVPDRFSGSGSGTDFTLTISSLQPEDF AVYYCQQHYITPLTFGAGTKVEIKRTVAAPSVFIFPPSDEQ LKSGTASVVCLLNNFYPREAKVQWKVDNALQSGNSQES VTEQDSKDSTYSLSSTLTLSKADYEKHKVYACEVTHQGLS SPVTKSFNRGEC | 10 |

The CDR definition scheme is: CDR definition rule: Kabat + Chothia

When p is equal to 1, the antibody-drug conjugate is generally referred to as "DAR1". When p is equal to 2, the antibody-drug conjugate is generally referred to as "DAR2". When p is equal to 3, the antibody-drug conjugate is generally referred to as "DAR3". When p is equal to 4, the antibody-drug conjugate is generally referred to as "DAR4", and so on.

## Compositions

The present disclosure also relates to a composition comprising one or more antibody-drug conjugates of formula (I) as defined above, or stereoisomers or pharmaceutically acceptable salts or solvates thereof. The composition may be a pharmaceutical composition comprising one or more antibody-drug conjugates of formula (I) as defined above, or stereoisomers or pharmaceutically acceptable salts or solvates thereof, and a pharmaceutically acceptable carrier.

The compositions according to the present disclosure have a particularly homogeneous character, which may lead to a better stability of the composition, a better efficacy and/or a reduction of side effects compared to non-homogeneous compositions.

Advantageously, when A is an antibody or antibody fragment (e.g., hRS7 ) targeting TROP2, the composition according to the present disclosure is characterized by the following features:

a) at least 50%, preferably at least 55%, at least 60%, or at least 65% (e.g., at least 70%, at least 75%, at least 80%, at least 85%, at least 90%, at least 95%, at least 96%, at least 97%, at least 98%, or at least 99%) of the antibody-drug conjugate or the stereoisomer thereof, or the pharmaceutically acceptable salt or solvate thereof in the composition has p equal to 1-15;

b) the average drug-to-antibody ratio (average DAR) is between 1 and 15; preferably between 1 and 10, between 2 and 8, or between 3 and 4. The average DAR is generally determined by HIC (hydrophobic interaction chromatography) method or by native mass spectrometry;

c) at least 95% (e.g., at least 96%, at least 97%, at least 98%, or at least 99%) of the antibody-drug conjugate exists in the form of a monomer. The percentage of monomer is generally determined by SEC (size exclusion chromatography) method;

or, d) a combination of two or three properties selected from a), b) and c).

Advantageously, when A is an antibody (e.g. hRS7 ), the composition according to the present disclosure is characterized by the following features:

a) at least 50%, preferably at least 55%, at least 60%, or at least 65% (e.g., at least 70%, at least 75%, at least 80%, at least 85%, at least 90%, at least 95%, at least 96%, at least 97%, at least 98%, or at least 99%) of the antibody-drug conjugates in the composition have a DAR value of 4;

b) an average drug-to-antibody ratio (average DAR) of 3.5 to 4.5, preferably 3.8 to 4.2, 3.9 to 4.1, or 3.9 to 4.0, e.g., equal to $4.0\pm0.2$ or $4.0\pm0.1$, e.g., equal to $3.93\pm0.01$. The average DAR is generally determined by HIC (hydrophobic interaction chromatography) method, native mass spectrometry, or high-performance liquid chromatography (e.g., RP-HPLC);

c) at least 95% (e.g., at least 96%, at least 97%, at least 98%, or at least 99%) of the antibody-drug conjugate exists in the form of a monomer. The percentage of monomer is generally determined by SEC (size exclusion chromatography) method;

or, d) a combination of two or three properties selected from a), b) and c).

Pharmaceutical combination and kit

In one aspect, the present invention also provides a pharmaceutical combination or a pharmaceutical combination product comprising the antibody-drug conjugate of formula (I) or (I'), the stereoisomer thereof, or the pharmaceutically acceptable salt or solvate thereof, and one or more additional therapeutic agents. Other therapeutic agents that can be combined or administered in combination with the molecule of the present invention encompass various therapeutic agents for treating tumors, such as chemotherapeutic agents, angiogenesis inhibitors, cytokines, cytotoxic agents, other antibodies, small molecule drugs, or immunomodulatory agents (e.g., immune checkpoint inhibitors or agonists).

In yet another aspect, the present disclosure relates to a kit for use in treating or preventing a TROP2-associated disease, such as cancer, comprising the antibody-drug conjugate of formula (I) or (I'), the stereoisomer thereof, or the pharmaceutically acceptable salt or solvate thereof as provided herein, or the pharmaceutical composition comprising the antibody-drug conjugate of formula (I) or (I'), the stereoisomer thereof, or the pharmaceutically acceptable salt or solvate thereof as provided herein, optionally a container, and optionally a package insert or label indicating treatment.

Therapeutic use, treatment method

The present disclosure also relates to use of the antibody-drug conjugate of formula (I) or (I'), or the stereoisomer thereof, or the pharmaceutically acceptable salt or solvate thereof according to the present disclosure, or the composition comprising the antibody-drug conjugate of formula (I) or (I'), or the stereoisomer thereof, or the pharmaceutically acceptable salt or solvate thereof according to the present disclosure, as a medicament, e.g., a medicament for treating or preventing a TROP2-associated disease, such as a cell proliferative disease or disorder, or inhibiting abnormal cell growth (e.g., cancer).

The cancer may be selected from adenocarcinoma, brain cancer, bladder cancer, breast cancer, cervical cancer, choriocarcinoma, CNS tumor, colon or colorectal cancer, diffuse intrinsic pontine glioma, endometrial cancer, esophageal cancer, Ewing's sarcoma, fallopian tube cancer, gallbladder cancer, gastric cancer, glioblastoma, head and neck cancer, hematological cancer, Hodgkin's lymphoma, kidney cancer, laryngeal cancer, nasopharyngeal cancer, leukemia, liver cancer, lung cancer, lymphoma, melanoma, Merkel cell carcinoma, mesothelioma, multiple myeloma, myelodysplastic syndrome, neuroblastoma, non-Hodgkin's lymphoma, osteosarcoma, pancreatic cancer, peritoneal cancer, prostate cancer, ovarian cancer, renal cancer, rhabdomyosarcoma, salivary gland cancer, sarcoma, skin cancer, small intestine cancer, squamous cell carcinoma, testicular cancer, thyroid cancer, uterine cancer, and Wilms' tumor.

The cancer may be selected from one or more of the following: lung cancer (non-small cell lung cancer cell, squamous cell carcinoma), pancreatic cancer, throat cancer, nasopharyngeal cancer, gastric cancer, bladder cancer, breast cancer, ovarian cancer, uterine cancer, and prostate cancer.

In some embodiments, the cancer is a TROP2-positive cancer.

In some embodiments, the TROP2-positive cancer refers to abnormal expression or activity of TROP2 in a subject having the cancer. In some embodiments, the subject (particularly an adult subject) has TROP2 expression. In some embodiments, the subject has (e.g., an elevated level of, e.g., nucleic acid or protein level or activity of) TROP2 (e.g., as compared to a healthy subject). In some embodiments, the biological sample (e.g., tumor cells or tumor tissue) of the subject has TROP2 (e.g., at an elevated level, e.g., at a nucleic acid or protein level or activity) (e.g., compared to a biological sample of a healthy subject (e.g., a corresponding tissue or cell in a healthy subject), or compared to TROP2 in an adjacent healthy tissue or cell of the subject).

In some embodiments, the TROP2-positive cancer refers to tumor cells expressing TROP2 in an individual with the cancers. In some embodiments, the tumor cells of the individual expresses TROP2, e.g., moderately or highly expresses TROP2. In some embodiments, a TROP2-positive tumor refers to the abnormal expression of TROP2 in tumor cells. In some embodiments, the abnormal expression of TROP2 refers to that the expression of TROP2 on a tumor cell is higher than the expression of TROP2 in a control cell (e.g., a healthy cell of a corresponding tissue of a healthy individual, or compared to a healthy cell adjacent to the tumor cell).

The antibody-drug conjugate, or the stereoisomer, the pharmaceutically acceptable salt, or the solvate thereof, or the composition according to the present disclosure is preferably formulated for parenteral administration, e.g., intravascular (intravenous or intra-arterial), intraperitoneal, or intramuscular administration. As used herein, the term "parenteral administration" means modes of administration other than enteral and topical administration, usually by injection, and includes, without limitation, intravascular administration, intravenous administration, intramuscular administration, intraarterial administration, intrathecal administration, intracapsular administration, intraorbital administration, intratumoral administration, intracardiac administration, intradermal administration, and intraperitoneal administration, administration by injection, administration by transtracheal perfusion, as well as subcutaneous administration, intraarticular administration, subcapsular administration, subarachnoid administration, intraspinal administration, and intrasternal administration. In the context of the present disclosure, preference is given to intravenous administration, for example by intravenous infusion.

The dosage of the antibody-drug conjugate administered to a subject in need thereof will vary depending on a number of factors, including, but not limited to, the route of administration, the type and severity of the condition being treated, the condition of the patient, the size of the patient, the age of the patient, and the like. A skilled artisan, based on their knowledge in the art, can readily determine desired dosage ranges based on these and other factors. Animal models or clinical trials can also be used to determine appropriate dosages. For example, a typical dose of the antibody-drug conjugate may be 0.3mg/kg, 0.6mg/kg, 1mg/kg, 2mg/kg, 3mg/kg, 5mg/kg, 6mg/kg, 7mg/kg, 8mg/kg, 20mg/kg, 30mg/kg, or more. Administration may be in a single dose or, more generally, in multiple doses. The administration regimen can include an initial loading dose followed by a maintenance dose (e.g., weekly, every two weeks, every three weeks, monthly, or longer). The duration of treatment may vary depending on the condition being treated and the subject.

The antibody-drug conjugates or compositions according to the present disclosure can be used in monotherapy or in combination with antibody drugs, other antibody-drug conjugates, small molecule drugs, and/or other chemotherapeutic drugs, such as drugs with recognized therapeutic benefit in the condition under consideration. For example, these drugs may include paclitaxel, docetaxel, doxorubicin, cyclophosphamide, an aromatase inhibitor (e.g., anastrozole), or an antibody for anti-cancer immunotherapy (e.g., an anti-PD1 antibody).

To prepare a pharmaceutical composition comprising the antibody or the fragment thereof, the antibody or the fragment thereof or the ADC according to the present disclosure may be mixed with a pharmaceutically acceptable carrier or excipient. The composition may further comprise one or more additional therapeutic agents useful in the treatment or prevention of cancer such as breast cancer, colorectal cancer, lung cancer, multiple myeloma, ovarian cancer, liver cancer, gastric cancer, pancreatic cancer, acute myeloid leukemia, chronic myeloid leukemia, osteosarcoma, squamous cell carcinoma, peripheral nerve sheath tumors, schwannoma, head and neck cancer, bladder cancer, esophageal cancer, Barrett's esophagus cancer, glioblastoma, soft tissue clear cell sarcoma, malignant mesothelioma, neurofibromatosis, kidney cancer, melanoma, prostate cancer, benign prostatic hypertrophy (BPH), gynecomastia, rhabdomyosarcoma, and endometriosis.

Formulations of therapeutic agents can be prepared by mixing with physiologically acceptable carriers, excipients or stabilizers, for example in the form of lyophilized powders, syrups, aqueous solutions, lotions or suspensions.

In one aspect, the present disclosure also relates to a method for treating or preventing a TROP2-related disease such as cancer in a subject, which comprises administering to the subject a therapeutically effective amount of the antibody-drug conjugate of formula (I) or (I') or the stereoisomer thereof, or the pharmaceutically acceptable salt or solvate thereof, or the composition comprising the antibody-drug conjugate of formula (I) or (I') or the stereoisomer thereof, or the pharmaceutically acceptable salt or solvate thereof.

The present disclosure also relates to a method for treating cancer in a subject, comprising administering to the subject a therapeutically effective amount of the antibody-drug conjugate according to formula (I) or (I') of the present disclosure or the composition comprising the antibody-drug conjugate according to formula (I) or (I') of the present disclosure.

In one aspect, the present disclosure further provides use of the antibody-drug conjugate of formula (I) or (I') or the stereoisomer thereof, or the pharmaceutically acceptable salt or solvate thereof according to the present disclosure, or the composition comprising the antibody-drug conjugate of formula (I) or (I') or the stereoisomer thereof, or the pharmaceutically acceptable salt or solvate thereof according to the present disclosure, in manufacture of a medicament for treating or preventing a TROP2-associated disease such as cancer.

In one aspect, the present disclosure also provides use of the antibody-drug conjugate of formula (I) or (I') or the stereoisomer thereof, or the pharmaceutically acceptable salt or solvate thereof according to the present disclosure, or the composition comprising the antibody-drug conjugate of formula (I) or (I') or the stereoisomer thereof, or the pharmaceutically acceptable salt or solvate thereof according to the present disclosure, as a medicament, for example, as a medicament for treating or preventing a TROP2-associated disease such as cancer.

It should be understood that the TROP2-associated disease or cancer is as defined herein.

Preparation method

The present specification also relates to a method for preparing the antibody-drug conjugate of formula (I) or (I') as defined above, which comprises:

(i) preparing a linker-payload conjugate having a structure of the following formula (III):

$$Z\text{-}E\text{-}Q'\text{-}L_1\text{-}D \text{ formula} \qquad \text{(III)};$$

wherein E, Q', $L_1$ and D are as defined for formula (I);
Z is as defined for Z', but Y' therein is

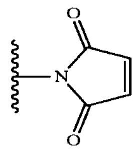

;

and

(ii) reacting the linker-payload conjugate obtained in step (i) with the TROP2-targeted antibody or antibody fragment as defined herein.

In some embodiments, before step (ii), the antibody or antibody fragment targeting TROP2 is treated with a reducing agent for thiol generation (TCEP), and the treatment is performed using one or more of the following: (i) the concentration of the antibody or antibody fragment targeting TROP2 is 2-10 mg/mL, e.g., the optimal concentration may be 5 mg/mL; (ii) the molar ratio of TCEP/mAb is 1.0 to 10.0 or 2.0-6.0, e.g., the optimal molar ratio is 2.0; (iii) the temperature is room temperature (20-37°C), and the optimal temperature is 25°C; (iv) the optimal pH value for the reaction is between 6.0 and 8.0; (v) the reaction time is 1-5h or 2-4h, e.g., 2h.

In some embodiments, the antibody or the antibody fragment targeting TROP2 is dissolved in a PBS buffer.

In some embodiments, in step (ii), the reduced TROP2-targeted antibody or antibody fragment is reacted with an excess of the linker-payload conjugate, and the reaction is performed using one or more of the following: (i) the molar ratio of MB3/mAb is 2.0-10.0 or 6.0-10.00, e.g., the optimal molar ratio is 6.0; (ii) the temperature is room temperature (20-37°C), and the optimal temperature is 25 °C; (iii) the reaction time is 1-5 h or 1-2 h, e.g., 2 h.

In a preferred embodiment, after step (ii), the resulting ADC crude product is purified, for example, by spin desalting, ultrafiltration, or dialysis, to obtain an ADC product.

In this case, the ADC product can be detected by RP-HPLC, LC-MS and SEC HPLC to determine the average DAR value, SEC purity and other parameters.

In some embodiments, the steps are performed under the specific reaction conditions disclosed in the examples.

It should be noted that embodiments in which the range or specific value of the specific reaction conditions disclosed in the examples varies by 100%, 80%, 60%, 40%, 20%, or 10% are also contemplated by the present invention.

DEFINITIONS

Unless defined otherwise, all technical and scientific terms used herein have the same meaning as commonly understood by one of ordinary skill in the art to which this application belongs. The following references are provided to give those skilled in the relevant art(s) many common definitions of terms used in the present disclosure: Singleton et al., Dictionary of Microbiology and Molecular Biology (2nd ed., 1994); the Cambridge Dictionary of Science and Technology (Walker ed., 1988); the Glossary of Genetics, 5th ed., R. Rieger et al. (eds.), Springer Verlag (1991); and Hale & Marham, The Harper Collins Dictionary of Biology (1991).

Unless otherwise explicitly indicated, the following terms used in the present disclosure shall have the following meanings described for them.

The term "alkyl" refers to a hydrocarbyl group of the general formula $C_nH_{2n+1}$. The alkyl group may be linear or branched. For example, the term "$C_{1-6}$ alkyl" refers to an alkyl group containing 1 to 6 carbon atoms (e.g., methyl, ethyl, n-propyl, isopropyl, n-butyl, isobutyl, sec-butyl, tert-butyl, n-pentyl, 1-methylbutyl, 2-methylbutyl, 3-methylbutyl, neopentyl, hexyl, 2-methylpentyl, etc.). Similarly, the alkyl moieties (i.e., alkyl) of alkoxy, alkylamino, dialkylamino, alkylsulfonyl, and alkylthio have the same definitions as above.

The term "alkylene" as used by itself or as part of another molecule refers to a divalent radical derived from an alkane. "Alkylene" is defined with reference to alkyl as defined above, but is divalent, i.e., has two single bonds to two other groups. For example, the group includes $-CH_2-$, $-CH_2CH_2-$, $-CH_2CH_2CH_2-$, $-CH_2CH_2CH_2CH_2-$, $-CH(-CH_2CH_3)-$ or $-CH_2CH(-CH_3)-$, all of which contain 10 or less carbon atoms, but the present disclosure is not limited thereto. The term "lower alkylene" refers to short alkylene groups, typically having 6 or fewer carbon atoms, including, for example, "$C_{1-6}$ alkylene".

The term "alkylene" as used in the specification, examples and claims, is intended to encompass both "unsubstituted alkylene" and "substituted alkylene". wherein the latter refers to an alkylene group having a substituent replacing a hydrogen atom on one or more carbon atoms of the hydrocarbon. Unless specifically stated otherwise, such substituents may include, for example, halogen, hydroxyl, carbonyl (e.g., carboxyl, alkoxycarbonyl, formyl, or acyl), thiocarbonyl (e.g., thioester, thioacetate, or thioformate), alkoxyl, phosphoryl, a phosphate group, a phosphonate group, a phosphinate group, an amino group, an amide group, an amidino group, an imine group, a cyano group, a nitro group, an azido group, a sulfhydryl group, an alkylthio group, a sulfate group, a sulfonic acid group, a sulfamoyl group, a sulfonamido

group, a sulfonyl group, a heterocyclyl group, an aralkyl group, or an aromatic or heteroaromatic group. When appropriately substituted, it will be understood by those skilled in the relevant art that the substituted residue on the hydrocarbon chain may itself be substituted. For example, the substituents of a substituted alkylene may include substituted and unsubstituted amino, azido, imino, amido, phosphoryl (including phosphonate and phosphinate), sulfonyl (including sulfate, sulfonamido, sulfamoyl, and sulfonate), and silyl groups, and may also include ether, alkylthio, carbonyl (including ketone, aldehyde, carboxylate, and ester), $-CF_3$, -CN, and equivalents thereof. Exemplary substituted alkyl groups are described below. The cycloalkylene may be further substituted with alkyl, alkenyl, alkoxy, alkylthio, aminoalkyl, carbonyl-substituted alkyl, $-CF_3$, -CN, and equivalents thereof.

The term "alkenyl" refers to a straight or branched chain hydrocarbon group containing from 2 to 16 carbon atoms and containing at least one double bond and no triple bonds. Alkenyl groups preferably contain 2-12 carbon atoms, 2-10 carbon atoms, 2-8 carbon atoms, 2-6 carbon atoms, or 2-4 carbon atoms. Representative examples of alkenyl include, but are not limited to, ethenyl, propenyl, butenyl, pentenyl, hexenyl, and the like.

The term "alkynyl" refers to a straight or branched chain hydrocarbon group containing from 2 to 16 carbon atoms and containing at least one triple bond. Alkynyl groups preferably contain 2-12 carbon atoms, 2-10 carbon atoms, 2-8 carbon atoms, 2-6 carbon atoms or 2-4 carbon atoms. Representative examples of alkynyl include, but are not limited to, ethynyl, propynyl, butynyl, pentynyl, hexynyl, and the like.

The term "halo" or "halogen" refers to fluorine, chlorine, bromine and iodine.

The term "haloalkyl" refers to an alkyl group, as defined herein, substituted with one or more halo groups, as defined herein. Haloalkyl may preferably be monohaloalkyl, dihaloalkyl or polyhaloalkyl (including perhaloalkyl). Monohaloalkyl groups may contain one iodo, bromo, chloro or fluoro in the alkyl. Dihaloalkyl and polyhaloalkyl groups may contain two or more of the same halo atoms or a combination of different halo groups in the alkyl group. Preferably, the polyhaloalkyl groups contain up to 12, 10 or 8 or 6 or 4 or 3 or 2 halogen groups. Non-limiting examples of haloalkyl include fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl, and dichloropropyl. A perhaloalkyl group refers to an alkyl group in which all of the hydrogen atoms are replaced with halogen atoms.

The term "haloalkenyl" refers to an alkenyl group, as defined herein, substituted with one or more halo groups, as defined herein. The term "haloalkynyl" refers to an alkynyl group, as defined herein, substituted with one or more halo groups, as defined herein. The meaning of "halo" defined with respect to "haloalkyl" is applicable to both "haloalkenyl" and "haloalkynyl".

The term "polyol group" refers to an alkyl group as defined above containing multiple (e.g., 2-10, e.g., 3, 4, 5, 6, 7, or 8) hydroxyl groups, optionally containing one or more (e.g., 2, 3, or 4) other groups (e.g., amino, carbonyl). Non-limiting examples of "polyol group" include, for example,

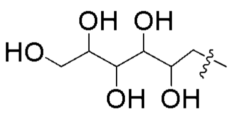 , 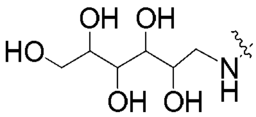 , 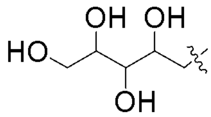 , or 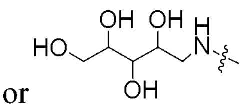 ,

wherein a chiral center without a stereo configuration indicated may be in R or S configuration, preferably

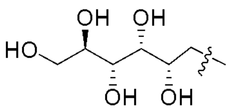 or 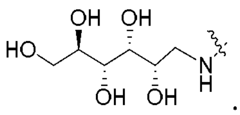 .

The term "amino acid" refers to naturally occurring and synthetic amino acids. The amino acids may be L or D isomers. The writing of conventional amino acids referred to herein follows conventional usage. See, e.g., Immunology-A Synthesis (2nd Edition, E. S. Golub and D. R. Gren, Eds., Sinauer Associates, Sunderland, Mass. (1991)), herein incorporated by reference. In addition, in the present disclosure, amino acids are generally represented by single-letter and three-letter abbreviations well known in the art. For example, the amino acid may be selected from the group consisting of phenylalanine (Phe; F), tyrosine (Tyr; Y), leucine (Leu; L), glycine (Gly; G), alanine (Ala; A), valine (Val; V), lysine (Lys; K), citrulline (Cit), serine (Ser; S), glutamic acid (Glu; E), aspartic acid (Asp; D), asparagine (Asn), isoleucine (Ile), arginine (Arg), proline (Pro), and glutamine (Gln).

The term "optional" or "optionally" means that the subsequently described event or circumstance occurs or does not occur, and that the description includes instances where said event or circumstance occurs and instances where it does not. For example, when a group or structure is "optionally substituted", the group or structure may or may not be substituted.

The term "pharmaceutically acceptable salt" refers to a salt that retains the biological effects and properties of the

ADCs of the present invention and is not biologically or otherwise undesirable. The ADCs of the present invention may be present in the form of their pharmaceutically acceptable salts, including acid addition salts and base addition salts. In the present invention, pharmaceutically acceptable non-toxic acid addition salts refer to salts formed by the ADCs of the present invention with organic or inorganic acids including, but not limited to, hydrochloric acid, sulfuric acid, hydrobromic acid, hydroiodic acid, phosphoric acid, nitric acid, perchloric acid, acetic acid, oxalic acid, maleic acid, fumaric acid, tartaric acid, benzenesulfonic acid, methanesulfonic acid, salicylic acid, succinic acid, citric acid, lactic acid, propionic acid, benzoic acid, p-toluenesulfonic acid, malic acid, and the like. Pharmaceutically acceptable non-toxic base addition salts refer to salts formed by the ADCs of the present invention with organic or inorganic bases, including, but not limited to, alkali metal salts, such as lithium, sodium or potassium salts; alkaline earth metal salts, such as calcium or magnesium salts; organic base salts, such as ammonium salts formed with organic bases containing N groups.

The term "solvate" refers to an association compound formed by one or more solvent molecules and the ADC antibody-drug conjugate in the present invention. Solvents for forming solvates include, but are not limited to, water, methanol, ethanol, isopropanol, ethyl acetate, tetrahydrofuran, N,N-dimethylformamide, dimethylsulfoxide, and the like.

"Pharmaceutically acceptable" and "pharmaceutically useful" are used interchangeably herein, where not inconsistent from the context.

In the present disclosure, the antibody-drug conjugate (formula (I) or (II)) of the present invention may have one or more chiral (asymmetric) centers. The present disclosure encompasses all stereoisomeric forms of the antibody-drug conjugate. Asymmetric centers can have, independently of one another, the (*R*) or (*S*) configuration. When bonds to chiral carbons are depicted as straight lines in the structural formulas of the present disclosure, or when a compound name is depicted without (*R*) or (*S*) chiral designation of the chiral carbon, it is to be understood that the (*R*) and (*S*) configurations of each such chiral carbon, and thus each enantiomer or diastereomer and mixtures thereof, are included in the formula or name. The production of particular stereoisomers or mixtures thereof may be identified in the examples where such stereoisomers or mixtures are obtained, but this in no way limits the inclusion of all stereoisomers and mixtures thereof within the scope of the present disclosure. When a bond at a chiral carbon is described as a solid line or a dashed line in the structural formulas of the present disclosure, or when a compound name is described in the context of a chiral (*R*) or (*S*) chiral name at a chiral carbon, it should be understood that the compound represented by the structural formula or name at that time has a determined stereoconfiguration at that chiral carbon position and will be distinguished from other stereoisomers or enantiomers or diastereomers or mixtures thereof.

The term "drug:antibody ratio", "drug-to-antibody ratio", or "DAR" refers to, for an antibody-drug conjugate molecule, the ratio of drug moieties (D) conjugated to the A (antibody) moieties described herein to the A moieties, i.e., the number of drug moieties (D) linked to each antibody (A). It should be understood that DAR may be determined by q in formula II. For example, DAR may be an integer from 1 to 16, e.g., an integer from 2 to 16, 4 to 16, 5 to 12, 6 to 10, 2 to 8, 3 to 8, 2 to 6, 4 to 6, or 6 to 10, e.g., 2, 3, 4, 5, 6, 7, 8, 9, 10, 11, or 12. For antibody-drug conjugate products, they are generally characterized by an average DAR, i.e., the overall ratio of the drug moiety (D) conjugated to moiety A described herein to moiety A in the product, as measured by detection methods (e.g., by conventional methods such as mass spectrometry, ELISA assay, electrophoresis, and/or HPLC), which is referred to herein as an average DAR or a measured DAR. The average DAR may be an integer or a decimal. In some embodiments, the average DAR value of the conjugate of the present invention is 1 to 16, e.g., 2-16, 4-16, 5-12, 6-10, 2-8, 3-8, 2-6, 3-5, 3.5-4.5, 4-6, or 6-10, e.g., 1.0-8.0 or 2.0-6.0, e.g., 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9.0, 9.1, 9.2, 9.3, 9.4, 9.5, 9.6, 9.7, 9.8, 9.9, or 10.0, and ranges having two of these values as endpoints. It should be understood that when referring to an average DAR value, the ADC of the present invention refers to a population of ADC molecules or a mixture of ADC molecules comprising ADC molecules with the same and/or different DARs.

The term "therapeutic agent" described herein encompasses any substance effective in preventing or treating a tumor, e.g., cancer, including chemotherapeutic agents, cytokines, angiogenesis inhibitors, cytotoxic agents, other antibodies, small molecule drugs, or immunomodulatory agents (e.g., immunosuppressive agents).

The term "cytotoxic agent" as used herein refers to a substance that inhibits or prevents cell function and/or causes cell death or destruction.

"Chemotherapeutic agents" include chemical compounds useful in the treatment of cancer or immune system diseases.

The term "drug" refers to an organic compound capable of modulating a biological process, in particular altering or preventing a pathological process.

The term "small molecule drug" refers to a low molecular weight organic compound capable of modulating a biological process, in particular altering or preventing a pathological process. "Small molecule" is defined as a molecule having a molecular weight of less than 10 kD, usually less than 2 kD and preferably less than 1 kD, more preferably less than 500 D. Small molecule drugs include, but are not limited to, organic molecules, organic molecules containing inorganic components, molecules containing radioactive atoms, synthetic molecules, peptidomimetics, and antibody

mimetics. As therapeutic agents, small molecules may be more permeable to cells, less susceptible to degradation, and less likely to elicit an immune response than large molecules.

The "drug (D)" moiety in the antibody-drug conjugate of the present invention may be substituted with isotopes including, but not limited to, e.g., deuterium, tritium, etc., which are referred to as deuterated compounds and tritiated compounds, respectively. For example, after a carbon-deuterium bond is substituted for a carbon-hydrogen bond, since the former is more stable than the latter, the substitution can directly affect properties such as absorption, distribution, metabolism, and excretion of certain drugs, thereby improving the efficacy, safety, and tolerability of the drugs. Therefore, the moiety "drug (D)" in the antibody-drug conjugate of the present disclosure may encompass compounds substituted with deuterium or tritium.

"Substituted with deuterium" means that hydrogens in the molecule are replaced with deuterium, for example, one or more hydrogens, e.g., 1-10 (e.g., 1, 2, 3, 4, 5, 6, 7, 8, 9, or 10) hydrogens, are replaced with deuterium.

As used herein, the term "comprise" or "include" is intended to mean that the described elements, integers or steps are included, but not to the exclusion of any other elements, integers or steps. If there are special explanations and instructions, they should be understood according to the special explanations and instructions. As used herein, the term "comprise" or "include", unless otherwise specified, also encompasses the situations composed of or consisting of the described elements, integers or steps. For example, when referring to an antibody variable region "comprising" a particular sequence, it is also intended to encompass an antibody variable domain consisting of that particular sequence.

The compounds and intermediates of the present disclosure may also exist in different tautomeric forms, and all such forms are included within the scope of the present disclosure. The term "tautomer" or "tautomeric form" refers to structural isomers of different energies which can interconvert via a low energy barrier. For example, proton tautomers (also known as prototropic tautomers) include interconversions via proton migration, such as keto-enol and imine-enamine isomerizations. A specific example of a proton tautomer is an imidazole moiety, where a proton can migrate between two ring nitrogens. Valence tautomers include interconversion by recombination of some bonding electrons.

As used herein, the term "antibody" is used in its broadest sense and includes immunoglobulins or other types of molecules that comprise one or more antigen-binding domains that specifically bind to an antigen, and is a protein or a polypeptide that exhibits binding specificity for a particular antigen. Specific examples of the antibody may include full-length antibodies (e.g., classical fourchain antibody molecules), single-chain antibodies, single-domain antibodies, multispecific antibodies, and the like. A classical antibody molecule is a heterotetramer consisting of two heavy chains of about 50-70 kDa each (heavy chains are referred to as H chains) and two light chains of about 25 kDa each (light chains are referred to as L chains) linked together by intra-chain and interchain disulfide bonds. Each chain consists of a variable region or a variable domain at the N-terminal position, called VL for the light chain and VH for the heavy chain, and a constant region at the C-terminal position of each chain, consisting of a single domain called CL in the light chain and three or four domains called CH1, CH2, CH3, CH4 in the heavy chain.

Antibodies can be divided into five major distinct classes, IgA, IgD, IgE, IgG, and IgM, based on the amino acid sequences of their heavy chain constant regions. These antibody classes can be further divided into subclasses, e.g., IgG1, IgG2a, IgG2b, IgG3, etc., according to the size of the hinge region, the position of the interchain disulfide bond, and the difference in molecular weight. Light chains can be divided into κ and λ classes according to the amino acid composition and arrangement of the light chain constant region of the antibody. The subunit structures and three-dimensional conformations of different classes of immunoglobulins are known in the art.

The term "chimeric antibody" is understood to mean an antibody in which the sequences of the light chain variable region and of the heavy chain variable region and of the light chain constant region and of the heavy chain constant region belong to different species. For the purposes of the present disclosure, the sequences of the heavy chain variable region and the light chain variable region are preferably derived from a murine source, while the sequences of the heavy chain constant region and the light chain constant region belong to a non-murine species. In this regard, for the constant region, all kinds of non-murine mammals can be used, in particular human, monkey, porcine, bovine, equine, feline, canine and even avian, which are listed not exhaustively. Preferably, the chimeric antibody according to the present disclosure comprises human heavy and light chain constant region sequences and murine heavy and light chain variable region sequences.

The term "humanized antibody" is understood to mean an antibody of which some amino acids of the sequences of all or some of the regions involved in antigen recognition (hypervariable regions or CDRs: complementarity determining regions) and sometimes some amino acids of the FR regions (framework regions) are of non-human origin, while the sequences of the constant and variable regions not involved in antigen recognition are of human origin.

The term "human antibody" is understood to refer to an antibody that comprises only human sequences for both the variable and constant regions of the light chain and the variable and constant regions of the heavy chain.

The term "antibody fragment" is understood to mean any part of an immunoglobulin obtained by enzymatic digestion or obtained by biological production, e.g. Fab, Fab', F(ab')2, Fab'-SH, scFv or scFv-Fc. In some embodiments, the antibody is an antigen-binding fragment, and the "antigen-binding fragment" of the antibody refers to an amino acid fragment in an antibody molecule that is involved in antigen-specific binding.

Enzymatic digestion of immunoglobulins by papain produces two identical fragments, which are referred to as Fab (antigen-binding fragment) fragment and Fc fragment (crystallizable fragment). Enzymatic digestion of immunoglobulins by pepsin yields Fc fragments and $F(ab')_2$ fragments that are cleaved into multiple peptides. $F(ab')_2$ consists of two Fab' fragments linked by interchain disulfide bonds. The Fab portion consists of a variable region and CH1 and CL domains. A Fab' fragment consists of a Fab region and a hinge region. Fab'-SH refers to Fab' fragments in which the cysteine residues of the hinge region bear a free thiol group. scFv (single chain variable fragment) is a fragment obtained from protein engineering, which consists of only VH and VL variable domains. The structure is stabilized by a short flexible peptide arm, called a linker, located between the two domains. The scFv fragment may be linked to an Fc fragment to form scFv-Fc.

The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable carrier" may be used in the sense of including an excipient, diluent or adjuvant. The carrier may, for example, be selected from the group consisting of lactose, dextrose, sucrose, sorbitol, mannitol, xylitol, erythritol, maltitol, starches, gum arabic, alginates, gelatin, calcium phosphate, calcium silicate, cellulose, methyl cellulose, polyvinylpyrrolidone, water, physiological saline, buffers such as PBS, methyl hydroxybenzoate, propyl hydroxybenzoate, talc, magnesium stearate and mineral oil. The carrier may include a filler, an anti-agglomeration agent, a lubricant, a wetting agent, a flavoring agent, an emulsifier, a preservative, or a combination thereof.

A "subject," "individual," or "patient" includes a vertebrate. Particularly preferred examples of animals include mammals, e.g., cattle, dogs, horses, cats, sheep, pigs, and primates (including humans and non-human primates), particularly humans. preferably refers to an animal in which a therapeutic or prophylactic effect can be achieved by the molecule of the present disclosure.

The term "treating" means administering the compound or formulation described herein to prevent, ameliorate, or eliminate a disease or one or more symptoms associated with the disease, and includes:

(i) preventing the occurrence of a disease or disease state in a mammal, particularly when such a mammal is predisposed to the disease state but has not yet been diagnosed as having it;
(ii) inhibiting a disease or disease state, i.e., arresting its development;
(iii) alleviating a disease or disease state, i.e., causing its regression.

The term "therapeutically effective amount" refers to an amount of the compound of the present disclosure for (i) treating or preventing a specific disease, condition or disorder; (ii) alleviating, ameliorating or eliminating one or more symptoms of a specific disease, condition or disorder, or (iii) preventing or delaying onset of one or more symptoms of a specific disease, condition or disorder described herein. The amount of the compound of the present disclosure constituting the "therapeutically effective amount" varies depending on the compound, the disease state and its severity, the mode of administration, and the age of the mammal to be treated, but can be determined routinely by those skilled in the art in accordance with their knowledge and the present disclosure.

The term "pharmaceutical combination" refers to a non-fixed combination product or a fixed combination product, including but not limited to a kit and a pharmaceutical composition. The term "non-fixed combination" means that the active ingredients (e.g., (i) the antibody-drug conjugate, or the stereoisomer or the pharmaceutically acceptable salt or solvate thereof, and (ii) an additional therapeutic agent) are administered to a patient as separate entities simultaneously, without specific time limitation, or sequentially at identical or different time intervals, wherein such administration provides prophylactically or therapeutically effective levels of two or more active agents in the patient. In some embodiments, the antibody-drug conjugate, the stereoisomer thereof, or the pharmaceutically acceptable salt or solvate thereof of the present invention and the additional therapeutic agent used in the pharmaceutical combination are administered at levels that do not exceed their levels when used alone. The term "fixed combination" means that two or more active agents are administered to a patient simultaneously in the form of a single entity. The dosages and/or time intervals for the two or more active agents are preferably selected so that the combined use of the parts will produce a greater effect in the treatment of the disease or condition than can be achieved by the use of either ingredient alone. The ingredients may each be in a separate formulation form, which may or may not be the same.

The term "combination therapy" or "combined administration" refers to the administration of two or more therapeutic agents or modalities (e.g., radiation therapy or surgery) to treat a disease described herein. Such administration includes co-administration of the therapeutic agents in a substantially simultaneous manner, e.g., in a single capsule having a fixed ratio of active ingredients. Alternatively, such administration includes co-administration of the active ingredients in a variety of or separate containers (e.g., tablets, capsules, powders, and liquids). The powder and/or liquid may be reconstituted or diluted to the desired dosage prior to administration. In addition, such administration also includes the use of each type of therapeutic agent in a sequential manner at approximately the same time or at different times. In

either case, the therapeutic regimen will provide the beneficial effect of the pharmaceutical combination in the treatment of a disorder or condition described herein.

The term "about" or "approximately" refers to an amount, level, value, number, frequency, percentage, dimension, size, component, weight, or length that varies by 30%, 25%, 20%, 25%, 10%, 9%, 8%, 7%, 6%, 5%, 4%, 3%, 2%, or 1% relative to the amount, level, value, number, frequency, percentage, dimension, size, component, weight, or length of the reference.

All patents, patent applications, and other identified publications are expressly incorporated herein by reference for the purpose of description and disclosure. Any reference to these publications herein shall not be construed as an admission that the publications form part of the commonly recognized knowledge in the art.

The compounds disclosed herein can be prepared by a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples disclosed herein.

## EXAMPLES

hTINA1 (the antibody used in Ds-1062) was a positive control antibody, and its sequence was derived from WHO Drug Information Vol. 34, No. 3, 2020. IgG was a negative control antibody from Equitech-bio, SLH56-0001.

Recombinant human Trop2 protein: 10428-H08H (Sino Biological); Rhesus Monkey Trop2 protein: 90893-C08H (Sino Biological); mouse TROP2: 50922-M08H (Sino Biological); rat TROP2: 86246-R08H (Sino Biological).

Trodelvy was a positive control ADC with Cat. No. information (GTIN (01) 0888818320019; LOT (10S22F010; serial (21) FWDED608C5Y2). The antibody moiety was hRS7, and the linker-payload was CL2A-SN38 (EMEA/H/C/005182/0000). The structure is as follows:

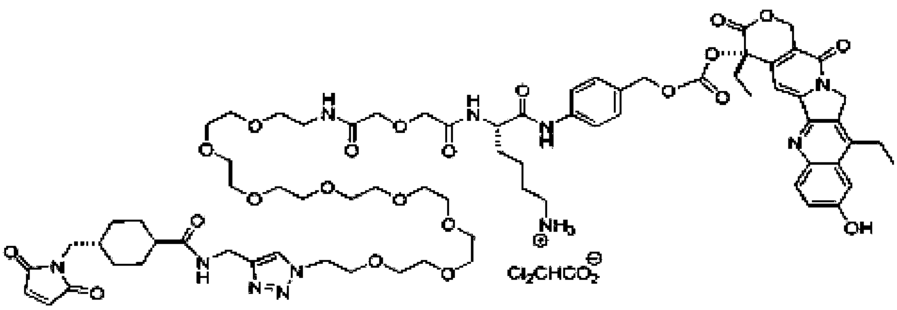

CL2A-SN38.

### Example 1. Preparation of Antibodies

Antibody preparation:

Expi293F cells (purchased from Gibco) were cultured in Expi293F medium (Gibco, REF# A14351-01). The cell density was measured the day before transfection (the viability should be greater than 95%). The cells were adjusted to $3 \times 10^6$ cells/mL with fresh Expi293F medium and cultured continuously. On the day of transfection, the cell density was adjusted to $3 \times 10^6$ cells/mL.

1/10 of the final transfection volume of Opti-MEM medium (Gibco, REF# 31985-070) was taken as a transfection buffer, and the DNA to be transfected was added at a ratio of 1 mg/L, wherein the ratio of light and heavy chain plasmids was 1:1. The mixture was mixed well. PEIMax (Polysciences Inc.,Cat# 24765-1) was added at the ratio of 1:3 of the DNA:PEI. The misture was mixed well, and incubated at room temperature for 20 min. Then the mixture was gently poured into the Expi293F cell suspension while shaking. The cells were cultured on a shaker at 120 rpm with 8% $CO_2$ at 36.5 °C.

After 16-18h of culture, the cell suspension was supplemented with 2% (by volume) of Feed (100 g/L Phytone Peptone + 100 g/L Difco Select Phytone) at a concentration of 200 g/L, a glucose solution at a final concentration of 5g/L, and Valproic acid sodium salt (Merk, Cat# P4543-100G) at a final concentration of 2.2mM. The mixture was gently mixed well, cultured at 8% $CO_2$, 36.5°C, and 120 rpm for another 7 days, and then collected. The cell feed liquid was well mixed with diatomite (Sartorius, Cat 1000037025) (40g of diatomite was added to 1 L of cell feed liquid), and the mixture was filtered through a 0.22μm disposable vacuum filtration device.

Purification of the proteins of interest by affinity chromatography:

A HiTrap MabSelect PrismA (GE Healthcare, Cat# 17549853) affinity chromatography column was selected for affinity capture. Before purification, 10-20 column volumes of 0.1 M NaOH was used to flow through the pipeline and the affinity chromatography column, then 10-20 column volumes of distilled water was used to wash the pipeline and the column, and 5 column volumes of 1× PBS (Gibco) was used to equilibrate the packing column. The filtered cell feed liquid was passed

through the column, and then 10 column volumes of 1× PBS was used to wash the packing column to remove non-specific binding proteins. The packing was washed with 5 column volumes of an elution buffer (100 mM sodium citrate, pH 3.5), and the eluate was collected, adjusted to pH 6.0 with 2 M Tris, filtered, and sterilized. After the purity was detected to be qualified, the antibody was used for ADC coupling.

**Example 2: Assay on Binding Activity of Anti-TROP-2 Antibodies to Antigen**

1. Determination of affinity (KD) of antibodies hRS7 and hTINA1 prepared in Example 1 for binding to TROP2 of different species

Affinity measurements were performed using bio-layer interferometry (BLI). Half an hour before the experiment, an appropriate number of AHC sensors (18-5060, Sartorius) were taken according to the number of samples and soaked in an SD buffer (1× PBS, 0.1% BSA, 0.05% Tween-20). The antibody hRS7 and human TROP2 (10428-H41H, Sino Biological), rhesus monkey TROP2 (90893-C08H, Sino Biological), mouse TROP2 (50922-M08H, Sino Biological), and rat TROP2 (86246-R08H, Sino Biological) were each diluted to 100 nM. SD buffer, antibody solution, human TROP2, rhesus monkey TROP2, mouse TROP2, and rat TROP2 were each added to a 96-well black polystyrene microplate (Greiner, 655209). Detection was performed using Fortebio Octet Red96e, and the sensor positions were selected according to the sample position layout. The instrument settings were as follows: the operation procedures were as follows: baseline equilibration for 120 s, sample loading for antibody immobilization for 100 s, baseline equilibration for 120 s, antigen binding for 100 s, and dissociation for 120 s, with a rotation speed of 1000 rpm and a temperature of 30 °C. After the experiment was completed, KD values were analyzed using ForteBio Octet analysis software. The results are shown in Table 2. As can be seen from the kinetic parameters of the affinity experiment, the binding affinity of hRS7 to human TROP2 and rhesus monkey TROP2 was 4.52 nM and 7.75 nM, respectively, and hRS7 did not bind to mouse TROP2 and rat TROP2 (data not shown).

Table 2: Affinity of antibodies hRS7 and hTINA1 for TROP2 of different species

| | KD-human | | | | KD - Rhesus monkey | | | |
|---|---|---|---|---|---|---|---|---|
| Sample Name | Response | KD (M) | Kon (1/Ms) | Kdis (1/s) | Response | KD (M) | Kon (1/Ms) | Kdis ( 1/s ) |
| hRS7 | 0.3603 | 4.52E-09 | 1.24E+0 5 | 5.60E-04 | 0.3137 | 7.75E-09 | 1.06E+0 5 | 8.23E -04 |
| hTIN A1 | 0.2839 | 2.28E-08 | 1.54E+0 5 | 3.50E-03 | 0.2102 | 3.25E-08 | 1.10E+0 5 | 3.56E -03 |

2. Testing the binding of TROP2 antibodies at the cellular level to human TROP2 protein

10% FBS (SH30406.05, HYCLONE) was added to an EMEM (30-2003, ATCC) medium and mixed well to prepare a FaDu cell (ATCC) growth medium. FaDu cells were adjusted to a concentration of $1\times10^{6}$ cells/mL with growth medium, plated into a 96-well plate (3799, Corning) at $1\times10^{5}$ cells/well, centrifuged, and washed twice with FACS buffer (1× PBS, 0.1% BSA, 0.05% Tween-20). The antibody was diluted with FACS buffer (a 3-fold serial dilution, starting from 200nM, a total of 9 concentrations, with the last concentration being 0 nM). The diluted antibody was resuspended in a plate with cells and stained at 4 °C for 30 min. After the staining was completed, the cells were washed twice with FACS buffer and then stained with a fluorescently labeled secondary antibody (410708, Biolegend) at room temperature for 20 min. After staining, the cells were washed twice with FACS buffer and detected by FACS (CytoFLEX, BECKMAN). The data were fitted by GraphPad Prism and the $IC_{50}$ of the drug was calculated (Table 3). Through testing, it was found that the expressed hRS7 antibody exhibited similar affinity to the control antibody hTINA1 (the antibody used in Ds-1062).

Table 3: Test results of binding ability of TROP2 antibodies to TROP2 protein on cell surface

| Antibody Name | $EC_{50}$ (nM) |
|---|---|
| hRS7 | 1.576 |
| hTINA1 | 1.696 |

**Example 3: Preparation of Compound MB3 and Linker-Dxd**

MB3 was prepared according to the method disclosed in Example 4 of WO2021/173773.

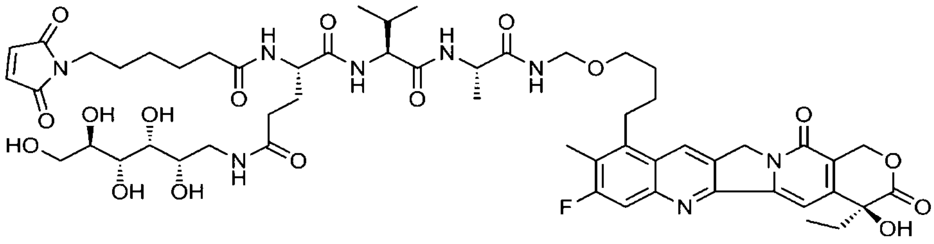

MB3.

Linker-Dxd represented by the following formula was prepared according to the method disclosed in Example 14 of WO2015/098099A1.

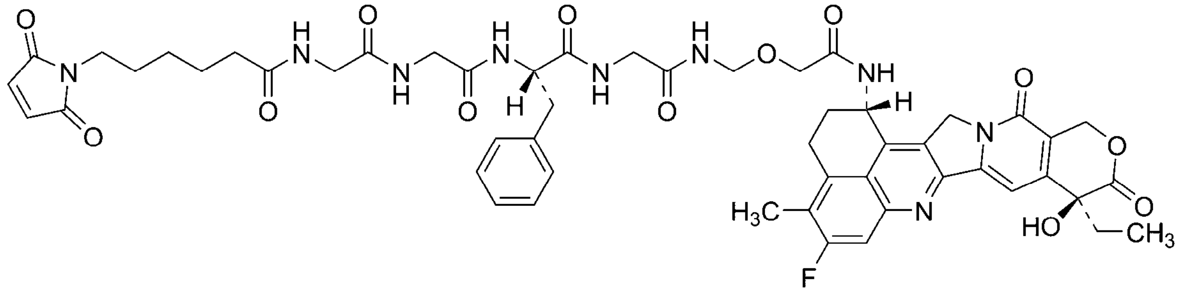

Linker-Dxd

## Example 4: Preparation of TROP2-Targeted Antibody-Drug Conjugate

### 4.1. Preparation of hRS7-MB3 according to the present disclosure

(a) antibody hRS7 was dissolved in PBS buffer (pH 7.4);
(b) a reducing agent solution (TCEP, Aldrich, Catalog Number 646547, dissolved in water) was added, and the reaction mixture was left to react at room temperature for 2 h, wherein: (i) the optimal concentration of hRS7 was 5 mg/mL, (ii) the optimal molar ratio of TCEP/mAb was 2.0, (iii) the optimal temperature for the reaction was 25 °C, and (iv) the optimal pH value for the reaction was between 6.0 and 8.0;
(c) an excess amount of MB3 (dissolved in DMSO) was added to react with the reduced antibody in step (a), and the reaction mixture was left to stand at room temperature for 2 h, wherein: (i) the optimal molar ratio of MB3/mAb was 6.0, and (ii) the optimal temperature for the reaction was 25 °C, thus obtaining a crude ADC product;
(d) subjecting the obtained ADC crude product to spin desalting, ultrafiltration, or dialysis purification to obtain a final ADC product;
(e) detecting the ADC product by RP-HPLC, LC-MS and SEC HPLC to determine the average DAR and SEC purity.

The average DAR value was 4.3, and the SEC purity was 98.42.

### 4.2 Preparation of hRS7-DXd and hTINA1-DXd

(a) antibody hRS7 or antibody hTINA1 was dissolved in PBS buffer (pH 7.4);
(b) a reducing agent solution (TCEP, Aldrich, Catalog Number 646547, dissolved in water) was added, and the reaction mixture was reacted at 0-10 °C for 12 h, wherein: (i) the optimal concentration of hTINA1 was 5 mg/mL, (ii) the optimal molar ratio of TCEP/mAb was 5.0, (iii) the optimal reaction temperature was 0-4 °C, and (iv) the optimal pH value for the reaction was between 6.0 and 8.0;
(c) an excess amount of Linker-DXd (dissolved in DMSO) prepared in Example 3 was added to react with the reduced antibody in step (a), and the reaction mixture was left to stand at 0-4 °C for 1-2 h, wherein: (i) the optimal molar ratio of DXd/mAb was 8, and (ii) the optimal temperature for the reaction was 0-4 °C, thereby obtaining a crude ADC product;
(d) subjecting the obtained ADC crude product to spin desalting, ultrafiltration, or dialysis purification to obtain a final

ADC product;

(e) detecting the ADC product by RP-HPLC, LC-MS and SEC HPLC to determine the average DAR and SEC purity.

The average DAR values of hRS7-DXd and hTINA1-DXd were 4.4 and 4.3, respectively, and the SEC purities were 99.29 and 97.59, respectively.

**Example 5. Preparation of IgG- Drug Conjugates**

**5.1 Preparation of IgG-MB3**

(a) Antibody IgG1 (isotype control) was dissolved in PBS buffer (pH 7.4);

(b) a reducing agent solution (TCEP, Aldrich, Catalog Number 646547, dissolved in water) was added, and the reaction mixture was left to react at room temperature for 2 h, wherein: (i) the optimal concentration of IgG1 (isotype control) was 10 mg/mL, (ii) the optimal molar ratio of TCEP/mAb was 2.0, (iii) the optimal temperature for the reaction was 25 °C, and (iv) the optimal pH value for the reaction was between 6.0 and 8.0;

(c) An excess amount of MB3 (dissolved in DMSO) was added to react with the reduced antibody in step (a), and the reaction mixture was left at room temperature for 2 h, wherein: (i) the optimal molar ratio of MB3/mAb was 6.0, and (ii) the optimal temperature for the reaction was 25 °C, thereby obtaining a crude ADC product.

(d) purifying the obtained ADC crude product by spin desalting, ultrafiltration, or dialysis to obtain a final ADC product;

(e) the ADC product was detected by RP-HPLC, LC-MS and SEC HPLC to determine the average DAR and SEC purity.

The average DAR value was 3.6, and the SEC purity was 99.34.

**5.2. Preparation of IgG-DXd**

(a) Antibody IgG1 (isotype control) was dissolved in PBS buffer;

(b) a reducing agent solution (TCEP, Aldrich, Catalog Number 646547, dissolved in water) was added, and the reaction mixture was left to react at room temperature for 2 h, wherein: (i) the optimal concentration of IgG1 (isotype control) was 10 mg/mL, (ii) the optimal molar ratio of TCEP/mAb was 2.0, (iii) the optimal temperature for the reaction was 25 °C, and (iv) the optimal pH value for the reaction was between 6.0 and 8.0;

(c) an excess amount of Linker-DXd (dissolved in DMSO) prepared in Example 3 was added to react with the reduced antibody in step (a), and the reaction mixture was left to stand at room temperature for 2 h, wherein: (i) the optimal molar ratio of Dxd/mAb was 6.0, and (ii) the optimal temperature for the reaction was 25 °C, thus obtaining a crude ADC product;

(d) subjecting the obtained ADC crude product to spin desalting, ultrafiltration, or dialysis purification to obtain a final ADC product;

(e) detecting the ADC product by RP-HPLC, LC-MS and SEC HPLC to determine the average DAR and SEC purity.

The average DAR value was 4.2, and the SEC purity was 98.69.

**Effect Example 1: In Vitro Efficacy Verification of TROP2-Targeted Antibody-Drug Conjugate (TROP2-Targeted ADC)**

1. In vitro cytotoxicity test of TROP2-targeted ADC

10% FBS (SH30406.05, HYCLONE) was added to RPMI 1640 (22400-071, Gibco), and the mixture was mixed well to prepare a BxPC3 cell (ATCC) growth medium. BxPC3 or FaDu cells were adjusted to a concentration of 50000 cells/mL with a growth medium, seeded into a 96-well white-bottom plate (167008, NUNC) at 50 μL per well (2500 cells/well), and incubated overnight in an incubator at 37 °C. The antibody was diluted with growth medium and serially diluted 5-fold from 200 nM (2×) to obtain 9 gradients, with the final concentration being 0 nM. The diluted antibody-drug conjugate or control was added at 50 μL/well, and the total volume was 100 μL. The edges of the well plate were sealed with 200 μL of PBS (pH 7.4), and the plate was incubated in an incubator at 37 °C for 6 days. The cultured 96-well plate and CTL (DD1101-02, Vazyme) were taken out and equilibrated to room temperature. An equal volume of CTL, i.e., 100 μL,

was added to each well. The cell pellet was fully lysed by shaking on a plate shaker for 5 min, and the luminescence signal was stabilized by standing at room temperature for 10 min. The culture plate was tested on a microplate reader (spectra MAX i3x, Molecular Divices), the A450 readings were taken, and the cell viability was calculated.

Table 4. **Experiment on in vitro cytotoxicity of TROP2-targeted ADC**

| | FaDu | BxPC3 |
|---|---|---|
| **TROP2-targeted ADC** | $IC_{50}$ (nM) | |
| hRS7-MB3 | 0.63 | 0.43 |
| hTINA1-DXd | 1.50 | 0.55 |
| Trodelvy | 0.53 | 0.42 |

The cell proliferation inhibition rate after 6 days of culture was calculated using the following formula.

Cell proliferation inhibition rate (%) = a/b $\times$ 100, where a: the average value of experimental sample-added wells after 6 days of culture; b: the average value of control medium-added wells after 6 days of culture. The data were fitted by GraphPad Prism and the $IC_{50}$ of the drug was calculated (Table 4).

It was found by testing that hRS7-MB3 showed a strong cytotoxic effect ($IC_{50}$ < 1 nM), which was stronger than that of the control molecule hTINA1-DXd. In human pharyngeal cancer squamous cell line FaDu, hRS7-MB3 exhibited the same level as hTINA1-DXd, and both were able to effectively inhibit the growth of tumor cells (FIG. 2A). Testing on the human pancreatic cancer cell line BxPC-3 showed that the tumor inhibitory effect of hRS7-MB3 was better than that of the control molecule hTINA1-DXd (FIG. 3A).

Compared with another positive control ADC molecule Trodelvy, hRS7-MB3 showed comparable effects on FaDu and BxPC-3 (Table 4), and both of them were able to potently kill tumor cells (FIGs. 2B and 3B). However, the average DAR value of the control molecule Trodelvy as a whole was about 7.6, which was about twice the average DAR value of the molecule hRS7-MB3 of the present disclosure. Therefore, it can be concluded that the ADC of the present disclosure has stronger cytotoxicity.

2 Bystander effect

An important challenge of antibody-drug conjugates (ADCs) in the treatment of solid tumors is the heterogeneous expression of target antigens in tumor tissues or metastases, i.e., cells with high, low, or no expression of target antigens may coexist, thereby affecting the efficacy of ADCs. The "bystander killing effect" is a possible way to solve this problem. The ADC can target cells with a high expression level of TROP2, kill target cells, and release loaded drugs. Some loaded drugs have good hydrophobic activity, and the released loaded drugs can pass through the cell membrane of cells around the target cell, thereby achieving the effect of further killing tumor cells.

To verify the bystander effect of the TROP2-targeted ADC, two kinds of cells, Colo-205 (Nanjing Cobioer Biosciences Co., Ltd.) and BxPC3 (ATCC), were used for this study. The medium used for both kinds of cells was RPMI 1640 (22400-071, Gibco) + 10% FBS (SH30406.05, HYCLONE). Colo-205 cells with a lower expression level of TROP2 were labeled with CellTrace™ Violet (C3455, Thermo) reagent and incubated at 37 °C for 20 min, and then a normal medium was added to terminate the reaction. The cells were washed twice with the medium. The prepared BxPC3 and Colo-205 cells were added to a 24-well plate (NEST) at a ratio of 3:1, and the TROP2-targeted ADC at a final concentration of 10 nM or an equal volume of medium was added to the corresponding wells, with IgG-MB3 as a negative control. The prepared samples were incubated in a 37 °C incubator for 5 days. After the culture was completed, the cells were collected and stained with LIVE/DEADTM (Invitrogen, L34975). The stained cells were detected by a flow cytometer.

After detection, it can be found that Colo-205 cells, which were originally not sensitive to the TROP2-targeted ADC, re-respond to the drug under the BxPC3-mediated "bystander effect". Compared with the control hTINA1-DXd, hRS7-MB3 produced a stronger bystander effect (FIG. 4), further demonstrating that the toxin payload of the TROP2-targeted ADC of the present disclosure has stronger hydrophobic properties, and can more effectively penetrate cell membranes and kill adjacent cells.

**Efficacy Example 2: In Vivo Efficacy Verification of Anti-TROP2 Antibody-Drug Conjugate**

1. Mice: immunodeficient mice CB17-SCID (Vital River) aged 6-8 weeks were placed in an SPF experimental environment for 3-5 days before the experiment to better adapt to the current living environment.

In all experiments, the long and short diameters of the tumor were measured twice a week using a vernier caliper, and the tumor volume ($mm^3$) was calculated from the measured data. The calculation formula is as follows:

$$\text{Tumor volume } (mm^3) = 0.5 \times \text{long diameter (mm)} \times \text{short diameter (mm)} \times \text{short diameter (mm)}$$

2. Human gastric cancer cell line NCI-N87 (ATCC) cells were resuspended in 1× PBS (Gibco, 10010049) at an appropriate concentration, and the tumor cells were implanted subcutaneously in the right abdomen of the mice at an inoculation amount of $2\times10^6$ /mouse. When the tumor volume reached 150-200 $mm^3$, the mice were divided into groups such that the average initial administration volume of each group was substantially the same. After grouping, different doses of antibody-drug conjugates hRs7-DXd, hRS7-MB3 and hTINA1-DXd, or control-drug conjugates IgG-MB3, IgG-DXd and IgG control were administered by intraperitoneal injection. After administration, it can be seen that compared with the control antibody-drug conjugates (IgG-MB3 and IgG-DXd), the two groups of TROP2-targeted antibody-drug conjugates hRS7-DXd, hTINA1-DXd and hRS7-MB3 at a low dose (1 mg/kg) all exhibited a certain degree of tumor inhibitory effect (FIG. 5A), and their tumor inhibitory effects were similar. In the high-dose group, hRS7-DXd and hTINA1-DXd exhibited comparable tumor inhibitory effects, indicating that the two TROP2 antibodies have equivalent endocytic activity. Compared with hRS7-DXd, hRS7-MB3 has a stronger tumor inhibitory effect. Finally, compared with TROP2-ADC (hTINA1-DXd) from Daiichi Sankyo, hRS7-MB3 at the same dose (3 mg/kg) exhibited a stronger and more significant tumor inhibitory effect (FIG. 5B), so it can be inferred that hRS7-MB3 may have the best activity in the same class.

3. Non-small cell lung cancer cell line HCC827 cells purchased from ATCC were inoculated subcutaneously into mice ($2\times10^6$ /mouse), and when the tumor volume reached 120-150 $mm^3$, the mice were grouped and administered. Different doses of the antibody-drug conjugate and the control drug were intraperitoneally injected into the mice, and the administration was performed only once throughout the experiment. The growth status of the mice was observed, and the changes in tumor volume of the mice were measured. After administration, it was found that the antibody-drug conjugate in the high dose group (5 mg/kg) exhibited a strong and equivalent tumor inhibitory effect (FIG. 6). In the low-dose group (2 mg/kg), both groups of TROP2-targeted ADCs exhibited tumor proliferation inhibition effects, but hRS7-MB3 showed a more significant tumor inhibition effect than hTINA1-DXd (DS-1062) (FIG. 6).

4. The lung squamous cell carcinoma cell line LK-2 (CBP60105) purchased from Nanjing Cobioer Biosciences was implanted subcutaneously in the right abdomen of the mice at an inoculation amount of $3 \times 10^6$ cells/mouse. When the tumor volume reached 150-200 $mm^3$, the mice were divided into groups such that the average initial administration volume of each group was substantially the same. The mice were administered only once throughout the experiment and injected intraperitoneally at a dose of 10 mg/kg. IgG-MB3 or IgG-DXd was used as a negative control. It was observed that two TROP2-targeted ADCs were also able to delay tumor growth in a tumor model with relatively low TROP2 expression (FIG. 7). Compared with hTINA1-DXd (DS-1062), hRS7-MB3 showed a better tumor inhibitory effect.

5. The pancreatic cancer cell line BxPC3 cells purchased from ATCC were implanted subcutaneously in the right abdomen of the mice at an inoculation amount of $3 \times 10^6$ cells/mouse. On day 8, when the tumor volume reached 150-180 $mm^3$, the mice were divided into groups, and the average initial administration volume of each group was ensured to be substantially the same. The mice after grouping were administered by intraperitoneal injection at doses of 3 mg/kg and 10 mg/kg, and the administration was performed only once throughout the experiment. The experimental group was administered with TROP2-targeted ADCs (hTINA1-DXd and hRS7-MB3), and the control group was treated with an equivalent dose of IgG-MB3 or IgG-DXd. As can be seen from the results (FIG. 8), hRS7-MB3 and hTINA1-DXd (DS-1062) exhibited equivalent tumor inhibitory effects at a low dose (3 mg/kg). In the high dose group (10 mg/kg), hRS7-MB3 showed a more significant tumor inhibitory effect than hTINA1-DXd. On day 38, the tumor volume of the hRS7-MB3 group was significantly lower than that of the hTINA1-DXd group (P=0.008). From the overall tumor growth curve, hRS7-MB3 at 3 mg/kg showed the same tumor inhibitory effect as hTINA1-DXd at 10 mg/kg, so it can be inferred that hRS7-MB3 may have better clinical performance than hTINA1-DXd.

6. The lung squamous cell carcinoma cell line EBC-1 (JCRB0820) cells purchased from the JCRB cell bank were implanted subcutaneously in the right abdomen of the mice at an inoculation amount of $1 \times 10^6$ /mouse. When the tumor volume reached 150-180 $mm^3$, the mice were divided into groups, and the average initial administration volume of each group was basically the same. The grouped mice were treated by intraperitoneal injection at a dose of 1 mg/kg, and only one administration was performed throughout the experiment. As shown in FIG. 9, at a low dose, hRS7-MB3 exhibited a very strong tumor inhibitory effect.

Trodelvy is an approved TROP2-ADC molecule, and its preclinical and clinical data show that the Linker-payload of Trodelvy has poor stability. Trodelvy is poorly stable in mice with a half-life of 11 hours (Bioconjugate Chem. 2015, 26, 919-931), leading to its clinical need for more frequent dosing (once a week) to maintain drug concentration in the blood.

Therefore, we inferred that in the in vivo mouse tumor model, a single administration of hRS7-MB3 had a better tumor inhibitory effect than a single administration of Trodelvy.

**Effect Example 3: ADC In Vivo Stability Assay in Mice**

1. The antibody-drug conjugate was injected into Balb/c mice (Vital River) via the tail vein at a dose of 10 mg/kg, and blood was collected at 0.083 h, 0.5 h, 2 h, 6 h, 24 h, 48 h, 96 h, 168 h, 336 h, and 504 h after administration (FIG. 10A). The blood samples were left to stand at room temperature and centrifuged to collect serum, so as to test the stability of the drug in the mice.
2. **Antibody detection**: TROP2 protein (ACRO, cat: TR2-H5223, lot: 2573-2316F1-1B9) was coated and incubated overnight at 4 °C (FIG. 10A). The coating protein solution was removed, and 300 μL of wash buffer (0.05% Tween 20) was added to each well for repeated washing 3 times. 200 μL of blocking solution (5% skim milk powder) was added to each well, and the plate was blocked at room temperature for 2 h. The blocking solution was discarded, and the plate was washed 3 times with 300 μL of wash buffer per well. The diluted standards, quality control samples, and mouse serum samples to be tested were added to the well plate and incubated at room temperature for 2 h. The sample solution was discarded, and 300 μL of wash buffer was added to each well for 3 times of repeated washing. Anti-hFc-HRP (BETHYL, cat:A80-104P, lot: 97) was diluted 20000-fold and added at 100 μL/well, and the plate was incubated at room temperature for 1 h. The liquid was removed, and 300 μL of wash buffer was added to each well for 6 times of repeated washing. 100 μL of TMB substrate (Solarbio, cat:PR1200, lot: 20230418) was added to each well, and the color development was performed at room temperature for 5-10 min in the dark. 50 μL of ELISA stop solution (Beijing Solarbio C1058 20190620) was added to each well, medium-speed shaking for 10 s, within 30 min, the OD values at 450 nm and 620 nm were read on a microplate reader (USA, Thermo Multiskan FC AS-A1-008).
3. **Antibody-drug conjugate ADC detection**: an anti-payload antibody 18E7C9 (innovent, lot: 20220424) was coated and incubated at 4 °C overnight (FIG. 10A). The coating protein solution was discarded, and 300 μL of wash buffer (0.05% Tween 20) was added to each well for 3 repeated washes. 200 μL of blocking solution (5% skim milk powder) was added to each well, and the plate was blocked at room temperature for 2 h. The blocking solution was discarded, and the plate was washed 3 times with 300 μL of wash buffer per well. The diluted standards, quality control samples, and mouse serum samples to be tested were added to the well plate and incubated at room temperature for 2 h. The sample solution was discarded, and 300 μL of wash buffer was added to each well for 3 times of repeated washing. Biotinylated TROP2 protein (ACRO, cat: TR2-H82E5, lot:BV3055-91PF1-ZC) was added to the microplate and incubated at room temperature for 2 h. The liquid was removed, and 300 μL of wash buffer was added to each well for 3 times of repeated washing. SA-HRP (Biolegend, cat:405210, lot: B339457) was diluted 20,000-fold and added at 100 μL/well, and the plate was incubated at room temperature for 1 h. The liquid was removed, and 300 μL of wash buffer was added to each well for 6 times of repeated washing. 100 μL of TMB substrate (Solarbio, cat:PR1200, lot: 20230418) was added to each well, and the color development was performed at room temperature for 5-10 min in the dark. 50 μL of ELISA stop solution (Beijing Solarbio C1058 20190620) was added to each well, medium-speed shaking for 10 s, within 30 min, the OD values at 450 nm and 620 nm were read on a microplate reader (USA, Thermo Multiskan FC AS-A1-008).

It was found by detection that the half-life (T-half) of hRS7-MB3 was 197.74 h, and compared with the half-life of the control ADC molecule hTINA1-DXd of 154.59 h (Table 5), hRS7-MB3 had better stability in mice as a whole. The overall exposure (AUC) of hRS7-MB3 in mice was also overall higher than that of hTINA1-DXd (Table 5, FIG. 10B). As can be seen from the clearance (Cl_obs) of the ADC overall molecule, the clearance of hRS7-MB3 was 0.59, which was much lower than that of the control molecule hTINA1-DXd (0.83) (Table 5). Compared with the half-life of the antibody, the linker-payload in hRS7-MB3 also had good stability (FIG. 10B). The half-life of the approved positive control ADC molecule Trodelvy in mice (10 mg/kg) was 11 h (Bioconjugate Chem. 2015, 26, 919-931), which was much lower than that of hRS7-MB3. In conclusion, it can be seen that hRS7-MB3 showed better stability in mice than the two positive control ADC molecules.

Table 5: Stability parameters of ADCs in mice

| | | T-half | AUC | Cl_obs |
|---|---|---|---|---|
| **Samples** | **Analysis Type** | **h** | **h*ug/ml** | **ml/h/kg** |
| **hRS7-MB3** | **ADC** | **197.74** | **17073.17** | **0.59** |
| **hRS7-MB3** | **Total-Ab** | **320.75** | **31096.81** | **0.32** |

(continued)

| | | T-half | AUC | Cl_obs |
|---|---|---|---|---|
| **Samples** | **Analysis Type** | **h** | **h*ug/ml** | **ml/h/kg** |
| **hTINA1-DXd** | **ADC** | **154.59** | **12053.60** | **0.83** |
| **hTINA1-DXd** | **Total-Ab** | **374.37** | **35805.22** | **0.28** |

**Effect Example 4: Safety Evaluation of Antibody-Drug Conjugate**

In order to evaluate the safety issues of antibody-drug conjugates, we selected cynomolgus monkeys, a species that cross-reacts with antibodies, for experiments in GLP toxicology. Specifically, the antibody-drug conjugate hRS7-MB3 was divided into three dose groups of 20 mg/kg, 50 mg/kg and 100 mg/kg, with 10 monkeys (5 males and 5 females) in each group, and administered at an interval of once every three weeks for a total of 3 times. After the end of the administration, the animals were continuously observed for 1-3 weeks. As a result, the highest non-severely toxic dose (HNSTD) of the antibody-drug conjugate hRS7-MB3 was 100 mg/kg.

In contrast, the HNSTD of the positive control ADC molecule DS-1062 (Daiichi Sankyo) was 30 mg/kg (WO2020240467A1) at the same dosing frequency (once every three weeks for a total of 3 times), which was much lower than that of hRS7-MB3. In addition, Trodelvy, another positive control ADC molecule, disclosed in the document (EMEA/H/C/005182/0000) an HNSTD (Days 1 and 8/every 21 days for 4 cycles) of 50 mg/kg. In conclusion, it can be seen that hRS7-MB3 has better tolerability in monkeys and may have a higher tolerated dose in clinical practice. Combined with the aforementioned results of in vivo and in vitro efficacy experiments, we can infer that hRS7-MB3 has a wider therapeutic window in clinical practice.

## Claims

1. An antibody-drug conjugate of the following formula (I), or a stereoisomer, a pharmaceutically acceptable salt or a solvate thereof:

$$A\left(Z'-E-Q'-L_1-D\right)_p$$ **formula (I),**

wherein D is shown as formula (II) below:

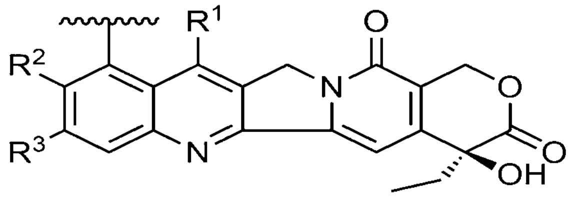

**Formula (II)**

wherein, $R^1$ is selected from H, $C_1$ -$C_6$ alkyl, $C_2$ -$C_6$ alkenyl, $C_2$ -$C_6$ alkynyl, $C_1$ -$C_6$ haloalkyl, $C_2$ -$C_6$ haloalkenyl and $C_2$ -$C_6$ haloalkynyl;
$R^2$ is selected from H, halogen, $C_1$ -$C_6$ alkyl, $C_1$ -$C_6$ haloalkyl, -$OR^6$ and -$SR^6$; $R^3$ is selected from H, halogen, CN, $C_1$ -$C_6$ alkyl, $C_1$ -$C_6$ haloalkyl and -$OR^6$; or $R^2$ and $R^3$ together form - $O(CH_2)_nO$- or -$O(CF_2)_nO$-, wherein n is 1 or 2;
$R^6$ is selected from H or $C_1$ -$C_4$ alkyl; and
$L_1$ is -$L_{1b}$-$L_{1a}$-, wherein $L_{1b}$ is linked to Q', and $L_{1a}$ is linked to D;

wherein $L_{1a}$ is absent or -($C_1$ -$C_{10}$ alkylene)-;
$L_{1b}$ is absent, *-($C_1$ -$C_{10}$ alkylene)-C(O)N($R^5$)- or *-($C_1$-$C_{10}$ alkylene)-N($R^5$)C(O)-; wherein * indicates that the end is covalently linked to Q'; and $R^5$ is H or $C_1$ -$C_6$ alkyl,

Q' is -O- or -S-;
E is -$CH_2$-NH-M, the M being a peptide residue comprising 2 to 10 amino acids; wherein the peptide residue is optionally substituted with one or more groups independently selected from the group consisting of $C_{1-6}$ alkyl and

a polyol group; and wherein the N-terminus of the M is covalently attached to Z';

Z' is -C(=O)-$L_2$-Y'-, wherein Z' is covalently linked to E through the -C(=O) moiety shown and linked to A through the Y' moiety;

$L_2$ is selected from $-(CH_2)_m-(O-CH_2CH_2)_{m1}-(CH_2)_{m2}-$, $-(CH_2)_m-(O-CH_2CH_2)_{m1}-NHC(O)-(CH_2)_m-$, and $-(CH_2)_m-(O-CH_2CH_2)_{m1}-C(O)NH-(CH_2)_m-$, wherein m1 and m2 are independently selected from integers of 0-20; m is selected from integers of 1-10, wherein the left end of these groups is linked to -C(=O)-, and the right end is linked to Y';

Y' is a group formed by

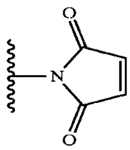

;

A represents an antibody or an antibody fragment targeting TROP2, comprising a heavy chain and a light chain, wherein the heavy chain comprises a CDR1 comprising or consisting of the sequence set forth in amino acid sequence SEQ ID NO: 1, a CDR2 comprising or consisting of the sequence set forth in amino acid sequence SEQ ID NO: 2, and a CDR3 comprising or consisting of the sequence set forth in amino acid sequence SEQ ID NO: 3; the light chain comprises a CDR1 comprising or consisting of the sequence set forth in amino acid sequence SEQ ID NO: 4, a CDR2 comprising or consisting of the sequence set forth in amino acid sequence SEQ ID NO: 5, and a CDR3 comprising or consisting of the sequence set forth in amino acid sequence SEQ ID NO: 6; and

p is the average value of the drug-to-antibody ratio and is a value between 1 and 15, e.g., a value between 3 and 5, or between 3.5 and 4.5.

**2.** The antibody-drug conjugate, or the stereoisomer, or the pharmaceutically acceptable salt or solvate thereof according to claim 1, wherein the antibody or the antibody fragment targeting TROP2 comprises a variable domain of the heavy chain and a variable domain of the light chain, wherein the variable domain of the heavy chain has at least 80% sequence identity, preferably at least 90% sequence identity, such as at least 95%, at least 96%, at least 97%, at least 98%, at least 99% or even 100% sequence identity, with the amino acid sequence SEQ ID NO: 7, and the variable domain of the light chain has at least 80% sequence identity, preferably at least 90% sequence identity, such as at least 95%, at least 96%, at least 97%, at least 98%, at least 99% or even 100% sequence identity, with the amino acid sequence SEQ ID NO: 8.

**3.** The antibody-drug conjugate, or the stereoisomer, or the pharmaceutically acceptable salt or solvate thereof according to claim 1 or 2, wherein the antibody or the antibody fragment targeting TROP2 comprises a variable domain of the heavy chain and a variable domain of the light chain, wherein the variable domain of the light chain comprises or consists of the amino acid sequence set forth in SEQ ID NO: 8, and the variable domain of the heavy chain comprises or consists of the amino acid sequence set forth in SEQ ID NO: 7.

**4.** The antibody-drug conjugate, or the stereoisomer, the pharmaceutically acceptable salt, or the solvate thereof according to any one of claims 1-3, wherein the antibody or the antibody fragment targeting TROP2 comprises a heavy chain and a light chain, wherein the light chain of the antibody or the fragment thereof targeting TROP2 has at least 80% sequence identity, preferably at least 90% sequence identity, such as at least 95%, at least 96%, at least 97%, at least 98%, at least 99%, or even 100% sequence identity, with the amino acid sequence SEQ ID NO: 10; and the heavy chain of the antibody or the fragment thereof targeting TROP2 has at least 80% sequence identity, preferably at least 90% sequence identity, such as at least 95%, at least 96%, at least 97%, at least 98%, at least 99%, or even 100% sequence identity, with the amino acid sequence SEQ ID NO: 9.

**5.** The antibody-drug conjugate, or the stereoisomer, or the pharmaceutically acceptable salt or solvate thereof according to any one of claims 1-4, wherein the antibody or the antibody fragment targeting TROP2 comprises a heavy chain and a light chain, wherein the heavy chain comprises or consists of the amino acid sequence set forth in SEQ ID NO: 9, and the light chain comprises or consists of the amino acid sequence set forth in SEQ ID NO: 10.

**6.** The antibody-drug conjugate, or the stereoisomer, the pharmaceutically acceptable salt, or the solvate thereof according to any one of claims 1-5, wherein the antibody fragment is selected from Fab, Fab', F(ab')2, Fab'-SH, scFv, or scFv-Fc.

**7.** The antibody-drug conjugate, or the stereoisomer, the pharmaceutically acceptable salt, or the solvate thereof according to any one of claims 1-6, wherein the antibody targeting TROP2 is a monoclonal antibody.

**8.** The antibody-drug conjugate, or the stereoisomer, the pharmaceutically acceptable salt, or the solvate thereof according to any one of claims 1-6, wherein the antibody targeting TROP2 is a chimeric antibody or a humanized antibody.

**9.** The antibody-drug conjugate or the stereoisomer thereof, or the pharmaceutically acceptable salt or solvate thereof according to any one of claims 1-8,

wherein, $R^1$ is selected from -H or $C_1$-$C_4$ alkyl; $R^2$ is selected from -H, -F, $C_1$ -$C_3$ alkyl or $C_1$ -$C_3$ haloalkyl; $R^3$ is selected from -H, -F, -CN, $-OCH_3$, $-CH_3$ or $-CF_3$;
$L_1$ is independently unsubstituted or halogenated -($C_1$ -$C_{10}$ alkylene)-;
Q' is -O- or -S-;
E is $-CH_2$-NH-M, the M being a peptide comprising 2 to 10 amino acids; wherein optionally the amino acids are substituted with one or more polyols; and wherein the N-terminus of the M is covalently attached to Z';
Z' is -C(=O)-$L_2$-Y';
$L_2$ is -($C_1$ -$C_{10}$ alkylene)-.

**10.** The antibody-drug conjugate, or the stereoisomer, the pharmaceutically acceptable salt, or the solvate thereof according to any one of claims 1-9, wherein Y' is

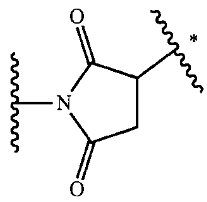

,

wherein * represents a site covalently linked to A.

**11.** The antibody-drug conjugate or the stereoisomer thereof, or the pharmaceutically acceptable salt or solvate thereof according to any one of claims 1-10,
wherein $L_2$ is $-(CH_2)_m-$, and m is selected from the group consisting of integers of 1-10.

**12.** The antibody-drug conjugate or the stereoisomer thereof, or the pharmaceutically acceptable salt or solvate thereof according to any one of claims 1-11, wherein Z' is formed by:

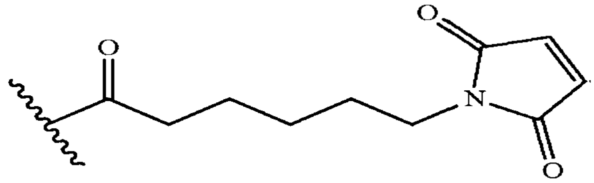

,

or

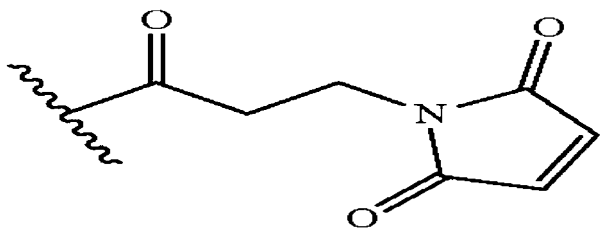

.

**13.** The antibody-drug conjugate or the stereoisomer thereof, or the pharmaceutically acceptable salt or solvate thereof

according to any one of claims 1-12, wherein Z' is:

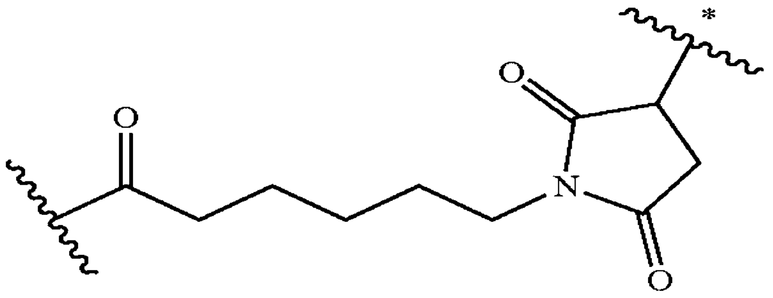

,

or

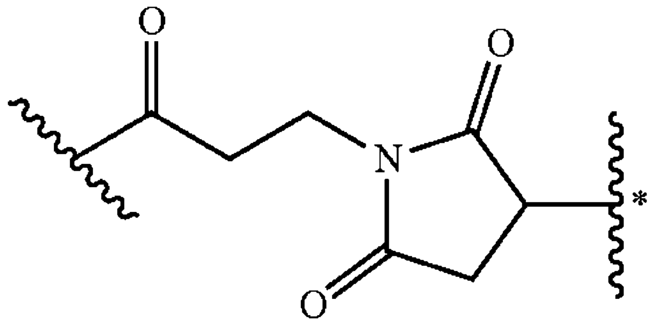

,

wherein * indicates the site of covalent attachment to A.

**14.** The antibody-drug conjugate, or the stereoisomer, the pharmaceutically acceptable salt, or the solvate thereof according to any one of claims 1-13, wherein M is a peptide comprising 2, 3, or 4 amino acids;

each amino acid of M is an L amino acid;
or at least one amino acid of M is a D amino acid.

**15.** The antibody-drug conjugate, or the stereoisomer, the pharmaceutically acceptable salt, or the solvate thereof according to any one of claims 1-13, wherein the amino acid is selected from the group consisting of glycine, alanine, valine, glutamine, glutamic acid, phenylalanine, leucine, tyrosine, lysine, citrulline, serine, tryptophan, aspartic acid, asparagine, isoleucine, arginine, and proline, and the glutamine or the glutamic acid is optionally substituted with one polyol group and optionally substituted with one $C_{1-6}$ alkyl.

**16.** The antibody-drug conjugate, or the stereoisomer, the pharmaceutically acceptable salt, or the solvate thereof according to any one of claims 1-15, wherein M comprises one or more amino acids selected from the group consisting of glycine, alanine, valine, glutamine, glutamic acid, phenylalanine, and leucine, and the glutamine or the glutamic acid is optionally substituted with a polyol.

**17.** The antibody-drug conjugate or the stereoisomer thereof, or the pharmaceutically acceptable salt or solvate thereof according to any one of claims 1-15,

the substituted glutamine or glutamic acid has the structure shown below:

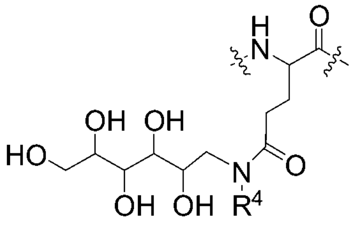 (G-1a), 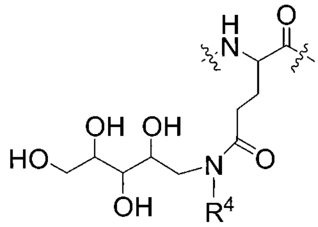 (G-1b),

wherein $R^4$ is H or $C_1$ -$C_6$ alkyl;
preferably

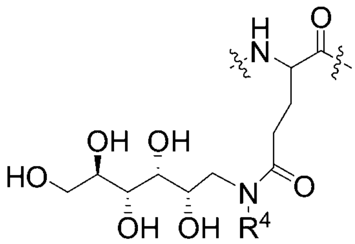 (G-1c)

wherein $R^4$ is H or $C_1$ -$C_6$ alkyl.

**18.** The antibody-drug conjugate or the stereoisomer thereof, or the pharmaceutically acceptable salt or solvate thereof according to claim 17,

the substituted glutamine or glutamic acid has the structure shown below:

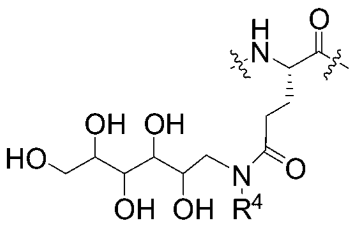 (G-2a), 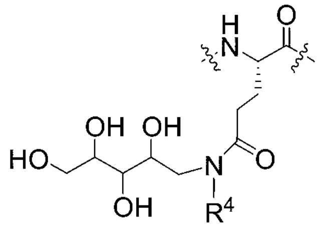 (G-2b),

wherein $R^4$ is H or $C_1$ -$C_6$ alkyl;
preferably

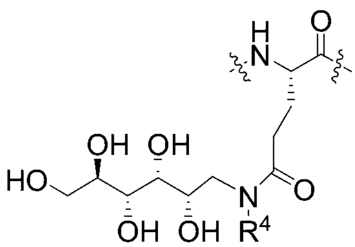 (G-2c)

wherein $R^4$ is H or $C_1$ -$C_6$ alkyl.

**19.** The antibody-drug conjugate or the stereoisomer thereof, or the pharmaceutically acceptable salt or solvate thereof according to any one of claims 1-17, wherein M comprises an amino acid having the following structure:

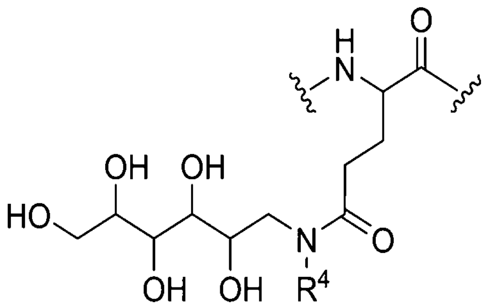

,

wherein $R^4$ is -H or $C_1$ -$C_6$ alkyl.

**20.** The antibody-drug conjugate or the stereoisomer thereof, or the pharmaceutically acceptable salt or solvate thereof according to claim 19, wherein M comprises an amino acid having the following structure:

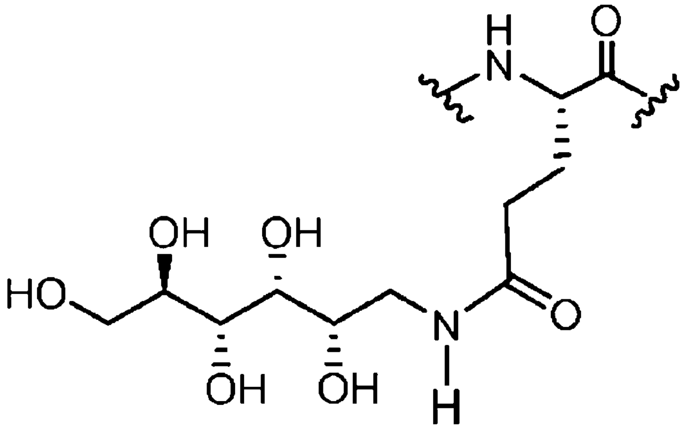

.

**21.** The antibody-drug conjugate or the stereoisomer thereof, or the pharmaceutically acceptable salt or solvate thereof according to any one of claims 1-20, wherein
M is selected from the group consisting of -Ala-Val-*, -Val-Ala-*, -Gly-Gly-*, -Leu-Ala-*, - Ala-Leu-*, -Ala-Ala-*, -Phe-Gln-*, -Gln-Phe-*, -Leu-Gln-*, -Gln-Leu-*, -Val-Gln-*, -Phe-Ala-*, - Ala-Phe-*, -Gln-Val-*, -Ala-Ala-Ala-*, -Gly-Gly-Gly-*, -Ala-Val-Ala-*, -Gly-Val-Gly-*, -Ala-Val-Gly-*, -Gly-Phe-Gly-*, -Lys-Phe-Gly-*, -Leu-Ala-Leu-*, -Val-Ala-Leu-*, -Leu-Ala-Val-*, -Val-Ala-Val-*, -Ala-Val-Gln-*, -Ala-Val-Ala-Gly-*, -Gly-Phe-Gly-Gly-*, -Gly-Gly-Phe-Gly-*, -Gly-Phe-Gly-Gln-*, -Ala-Val-Gly-Gly-*, -Ala-Ala-Ala-Ala-*, -Ala-Val-Ala-Ala-*, -Ala-Leu-Ala-Leu-*, -Leu-Ala-Leu-Ala-*, -Gly-Phe-Leu-Gly*, -Gly-Phe-Gly-Gln-*, and -Gly-Leu-Phe-Gly-*, wherein * indicates the N-terminus of the peptide covalently attached to Z'.

**22.** The antibody-drug conjugate or the stereoisomer thereof, or the pharmaceutically acceptable salt or solvate thereof according to any one of claims 1-20, wherein
M is selected from -L-Ala-D-Val-*, -L-Val-D-Ala-*, -L-Ala-L-Val-*, -L-Ala-D-Ala-*, -L-Ala-D-Ala-L-Ala-*, -L-Ala-L-Ala-L-Ala-*, -L-Ala-D-Val-L-Ala-*, -L-Ala-D-Ala-Gly-*, -L-Ala-D-Val-Gly-*, -L-Ala-L-Val-Gly-Gly-*, -L-Ala-L-Val-L-Gln-*, or -Gly-L-Phe-Gly-L-Gln-*, wherein * indicates the N-terminus of the peptide covalently attached to Z'.

**23.** The antibody-drug conjugate, or the stereoisomer, the pharmaceutically acceptable salt, or the solvate thereof according to any one of claims 1-22, wherein -E- is selected from the following structures, wherein * represents the N-terminus of the peptide covalently attached to Z':

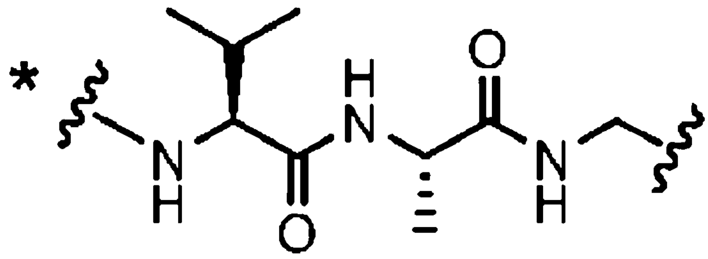

,

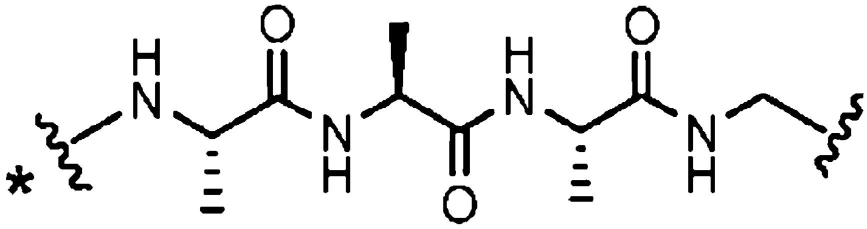

,

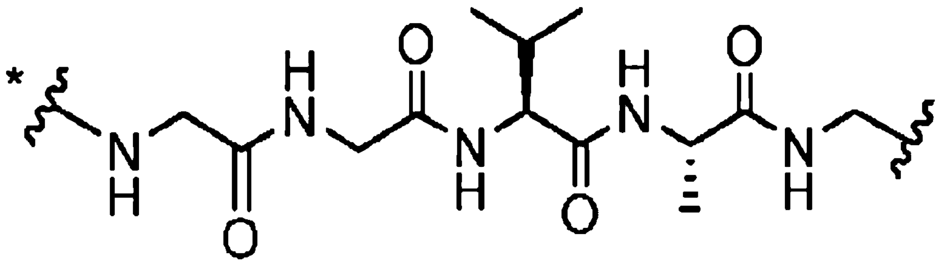

,

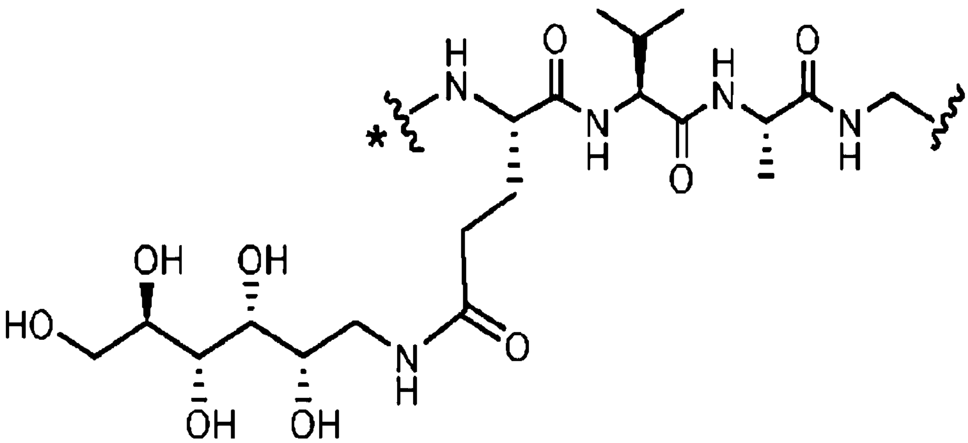

,

or

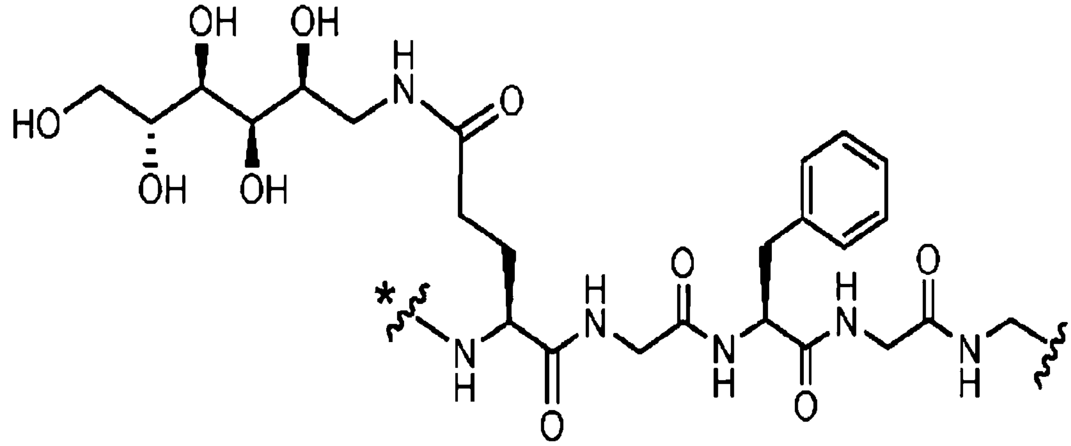

.

**24.** The antibody-drug conjugate or the stereoisomer thereof, or the pharmaceutically acceptable salt or solvate thereof according to claim 1, wherein Z'-E is formed of one of the following structures:

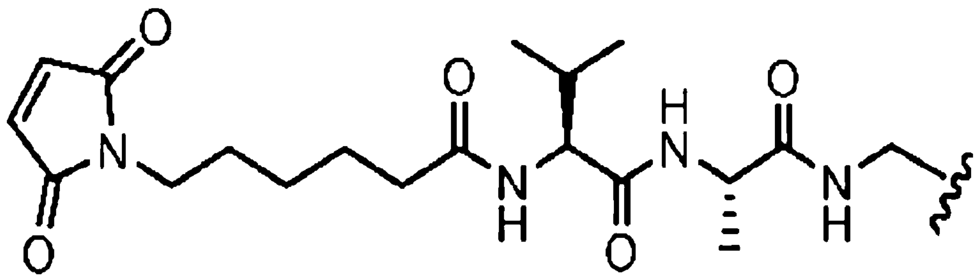

,

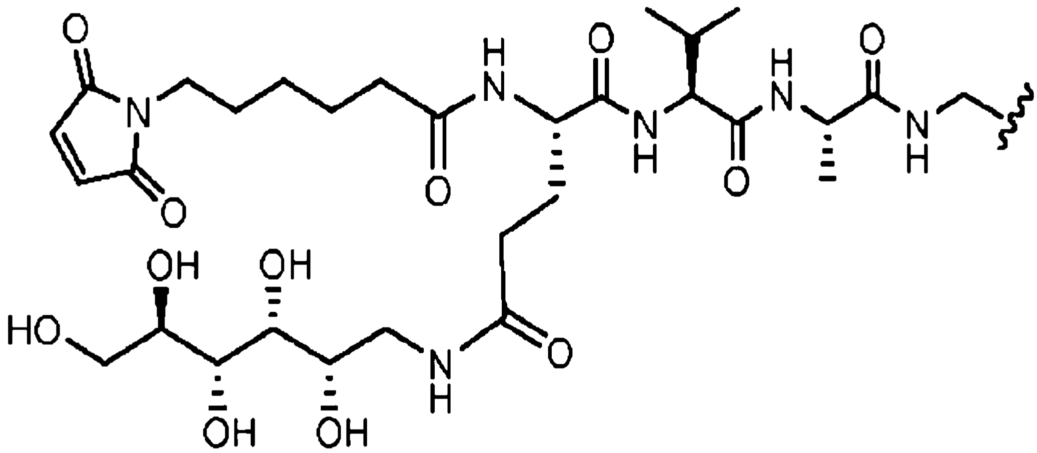

,

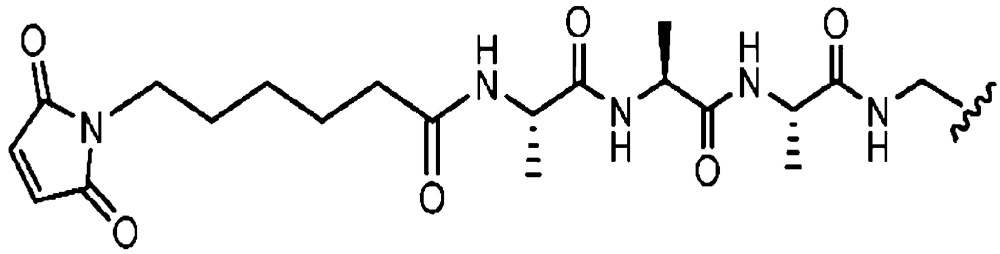

,

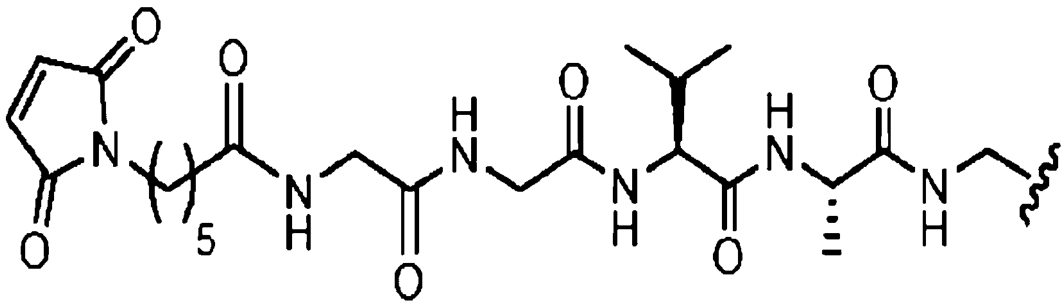

,

or

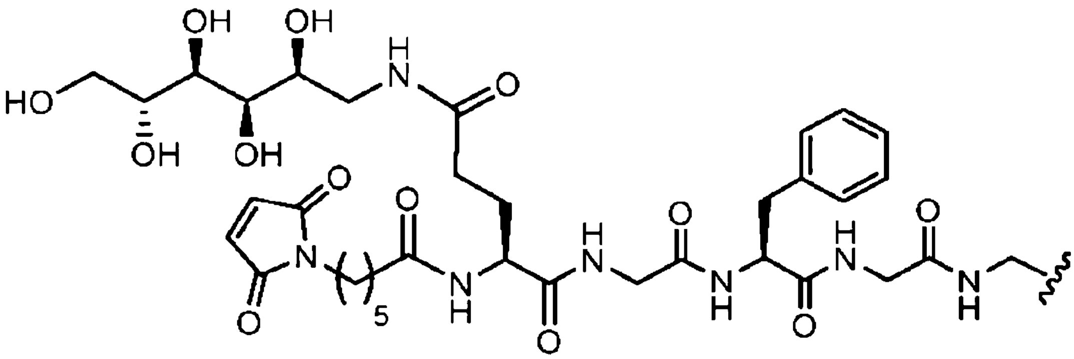

;

or, Z'-E is selected from the following structures, wherein * indicates a point of attachment to A:

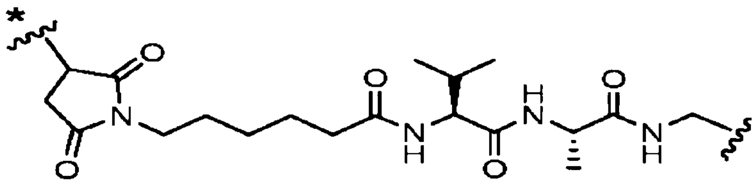

,

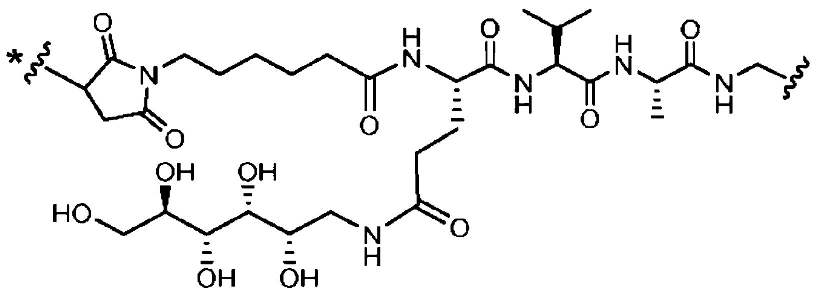

,

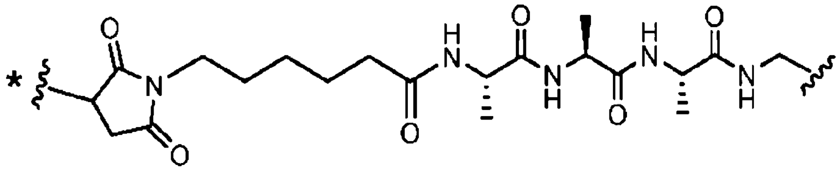

,

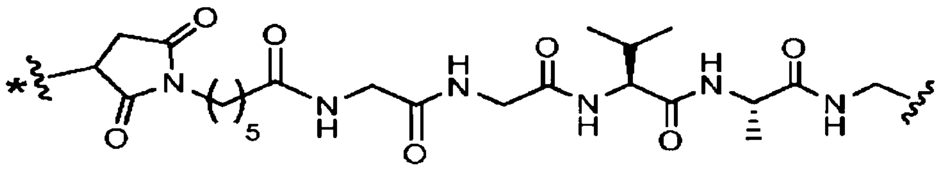

,

or

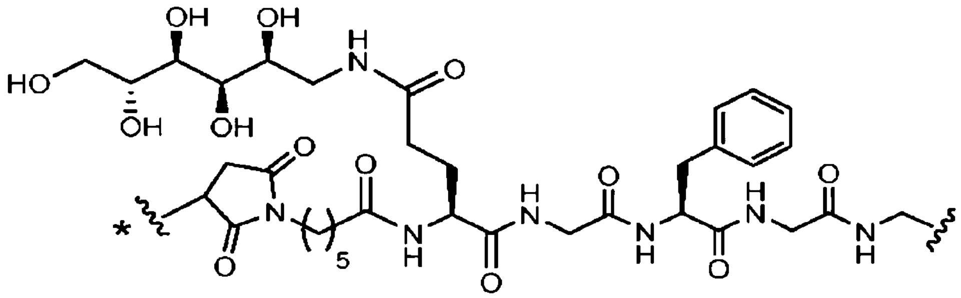

.

**25.** The antibody-drug conjugate or the stereoisomer thereof, or the pharmaceutically acceptable salt or solvate thereof according to any one of claims 1-24, wherein D is represented by the following structure:

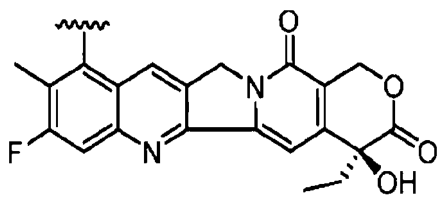

.

**26.** The antibody-drug conjugate or the stereoisomer thereof, or the pharmaceutically acceptable salt or solvate thereof according to any one of claims 1-25, wherein D-$L_1$ -Q'- has one of the following structures:

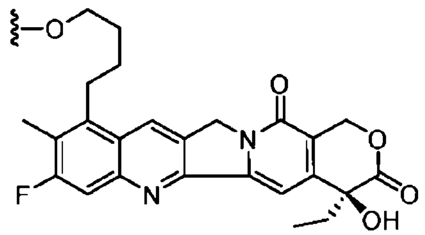

or

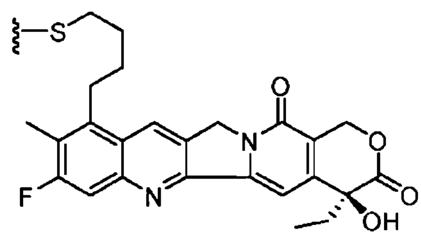

.

**27.** The antibody-drug conjugate or the stereoisomer thereof, or the pharmaceutically acceptable salt or solvate thereof according to any one of claims 1-26, wherein the antibody-drug conjugate has the following structure:

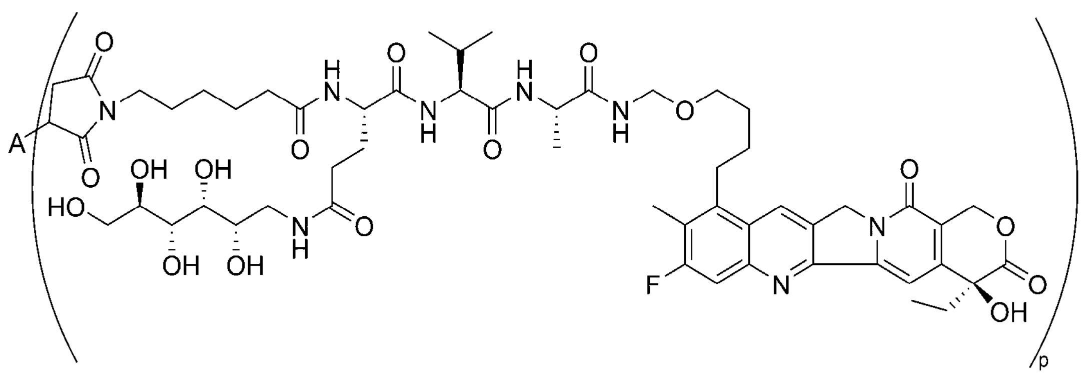

wherein A and p are as defined in one of claims 1 to 26.

**28.** An antibody-drug conjugate represented by formula (I'), or a stereoisomer or a pharmaceutically acceptable salt or solvate thereof,

$$A\left(Z'-E-Q'-L_1-D\right)_q \qquad (I')$$

wherein q is DAR, and q is 1, 2, 3, 4, 5, 6, 7, 8, 9, 10, 11, 12, 13, 14, or 15; and
A, Z', E, Q', $L_1$ and D are as defined in any one of claims 1-27.

**29.** A pharmaceutical composition, comprising the stereoisomer, pharmaceutically acceptable salt, or solvate thereof according to any one of claims 1-28, and optionally a pharmaceutically acceptable carrier.

**30.** A pharmaceutical combination comprising the stereoisomer, or the pharmaceutically acceptable salt or solvate thereof according to any one of claims 1-28, and one or more additional therapeutic agents, such as a chemotherapeutic agent, an angiogenesis inhibitor, a cytokine, a cytotoxic agent, an additional antibody, a small molecule drug, or an immunomodulatory agent.

**31.** Use of the antibody-drug conjugate or the stereoisomer thereof, or the pharmaceutically acceptable salt or solvate thereof according to any one of claims 1-28, the pharmaceutical composition according to claim 29, or the pharmaceutical combination according to claim 30 in the manufacture of a medicament for treating or preventing a cell proliferative disease or disorder or inhibiting abnormal cell growth, wherein preferably, the cell proliferative disease or disorder is cancer.

**32.** The use according to claim 31, wherein the cancer is adenocarcinoma, brain cancer, bladder cancer, breast cancer, cervical cancer, choriocarcinoma, CNS tumor, colon or colorectal cancer, diffuse intrinsic pontine glioma, endometrial cancer, esophageal cancer, Ewing's sarcoma, fallopian tube cancer, gallbladder cancer, gastric cancer, glioblastoma, head and neck cancer, hematological cancer, Hodgkin's lymphoma, kidney cancer, laryngeal cancer, nasopharyngeal cancer, leukemia, liver cancer, lung cancer, lymphoma, melanoma, Merkel cell carcinoma, mesothelioma, multiple myeloma, myelodysplastic syndrome, neuroblastoma, non-Hodgkin's lymphoma, osteosarcoma, pancreatic cancer, peritoneal cancer, prostate cancer, ovarian cancer, rhabdomyosarcoma, salivary gland cancer, sarcoma, skin cancer, small intestine cancer, squamous cell carcinoma, testicular cancer, thyroid cancer, uterine cancer, or Wilms' tumor; preferably, the cancer is a TROP2-positive cancer.

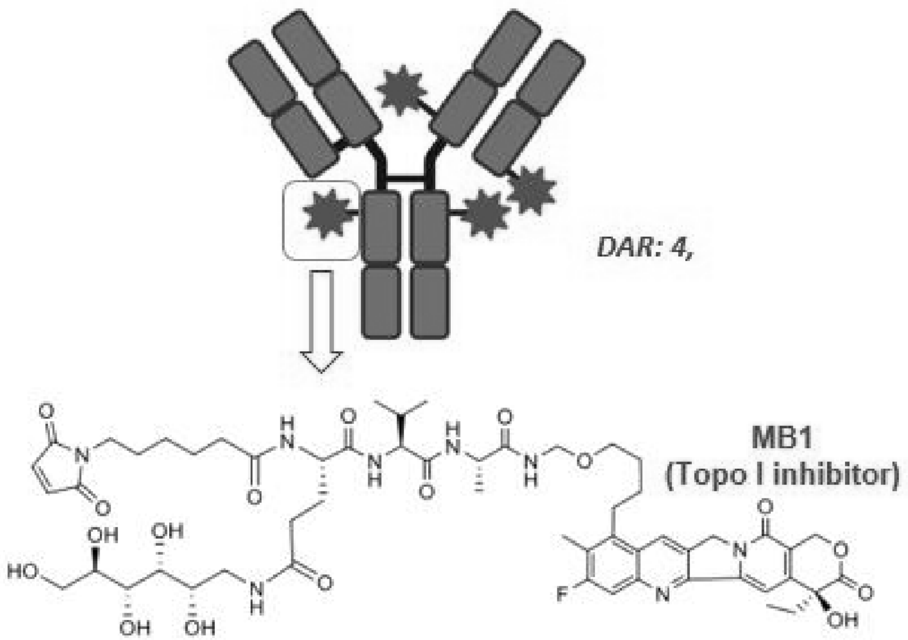


Figure 1

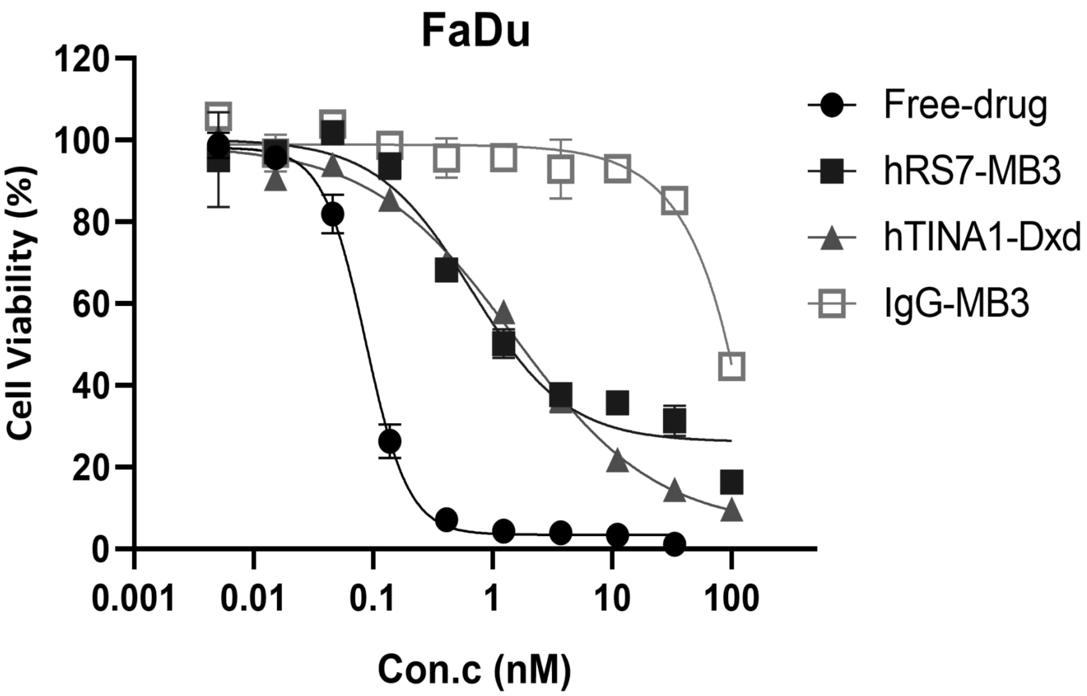


Figure 2A

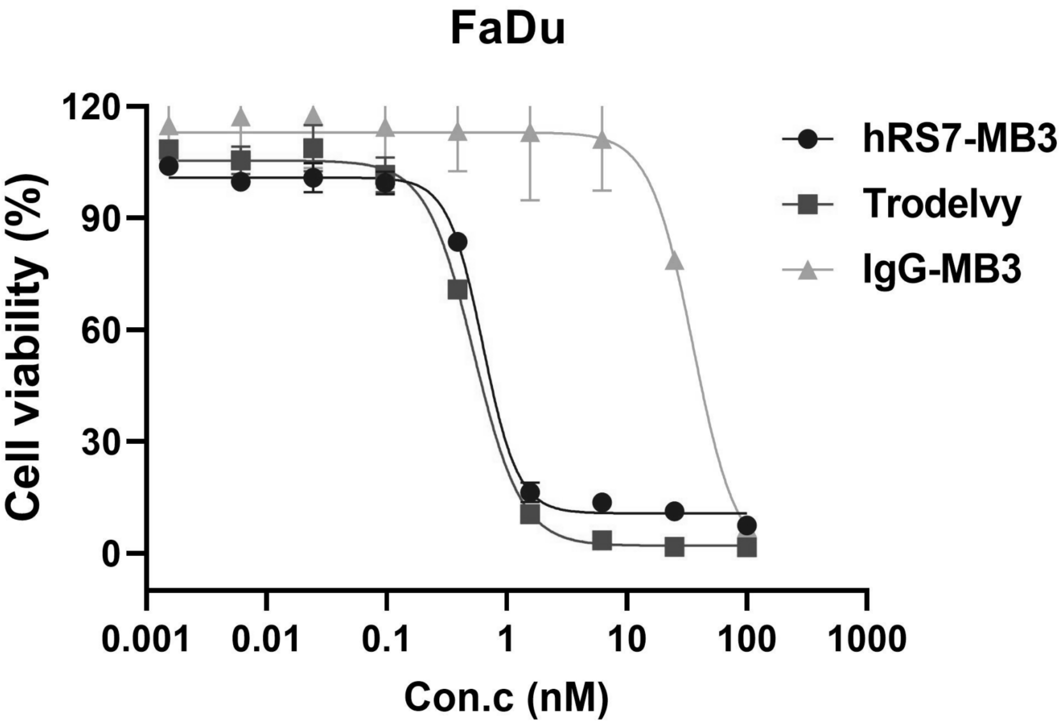


Figure 2B

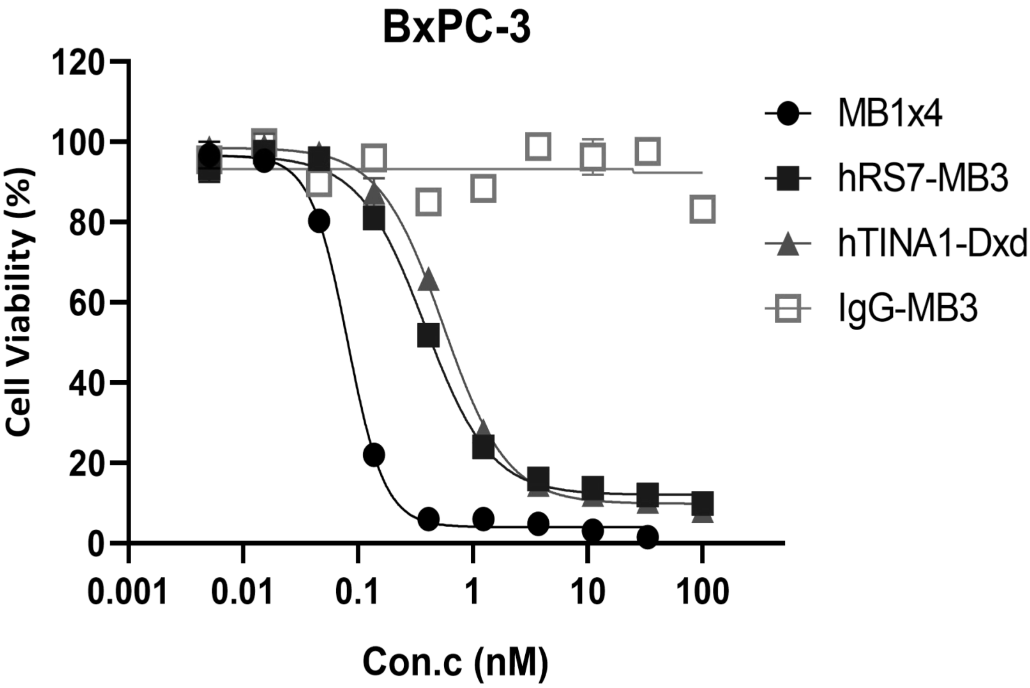


Figure 3A

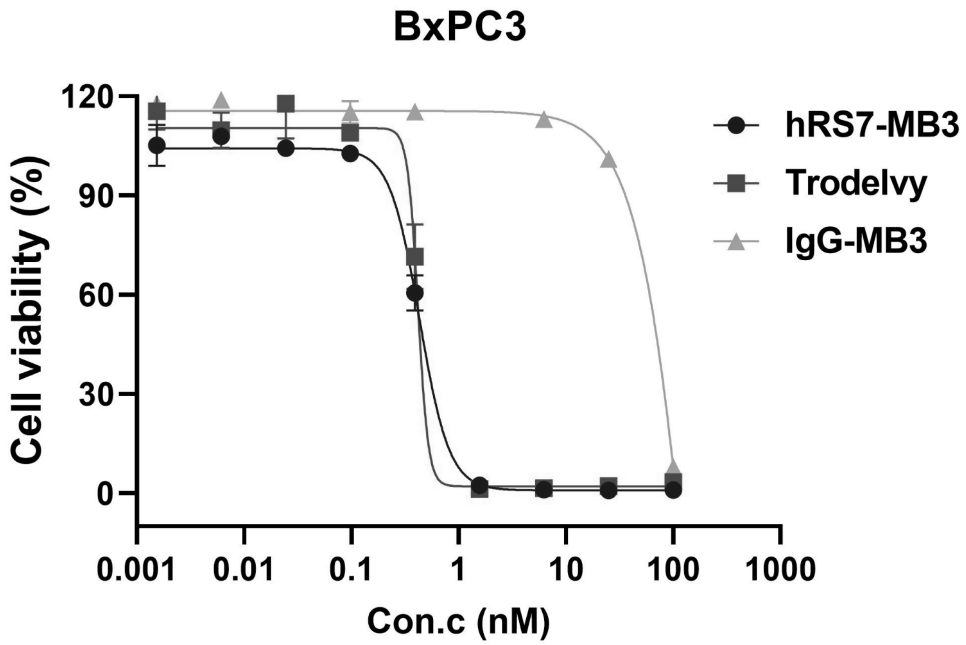


Figure 3B

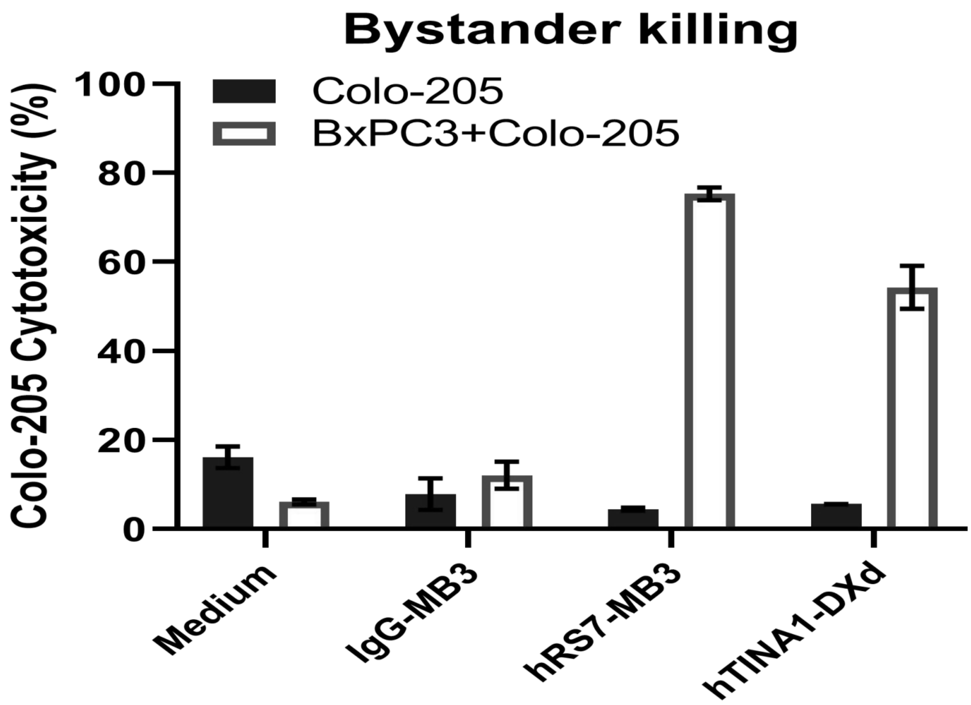


Figure 4

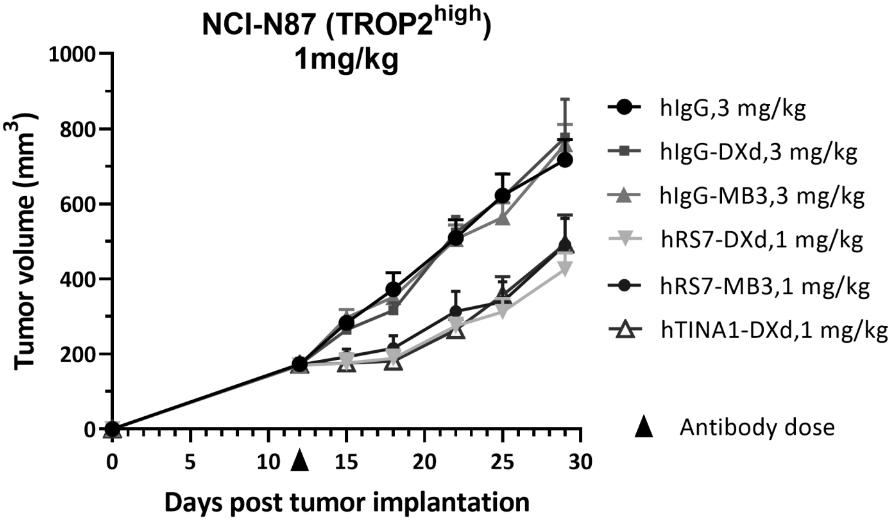


Figure 5A

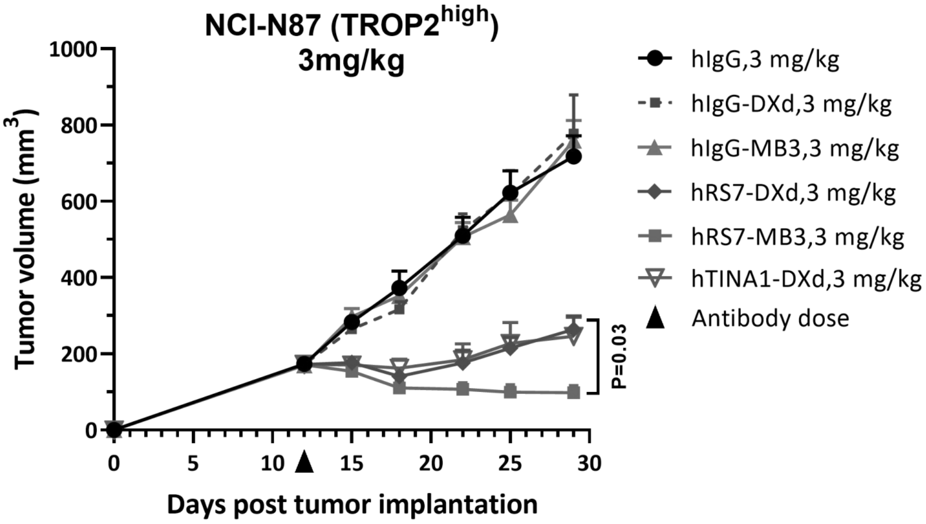


Figure 5B

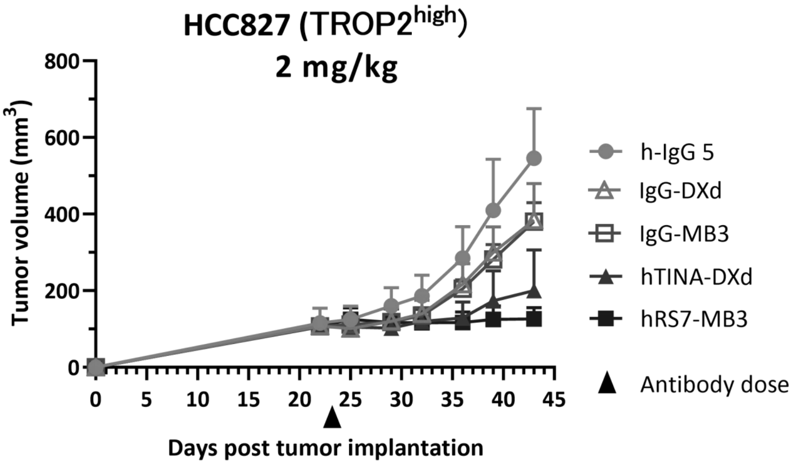


A

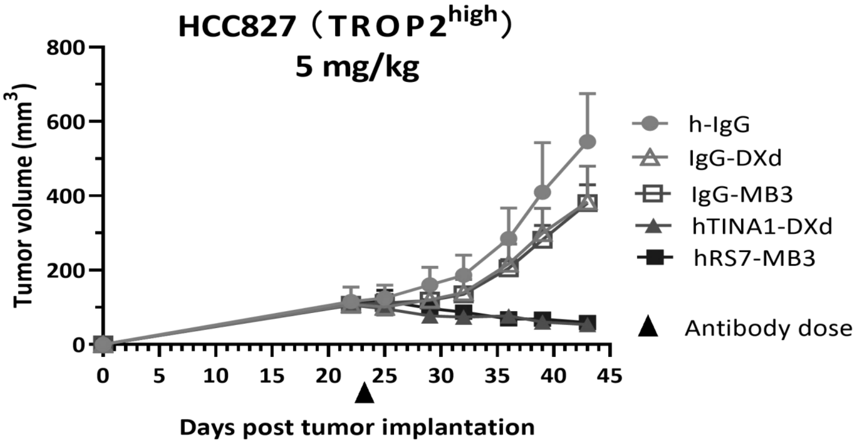


B

Figure 6.

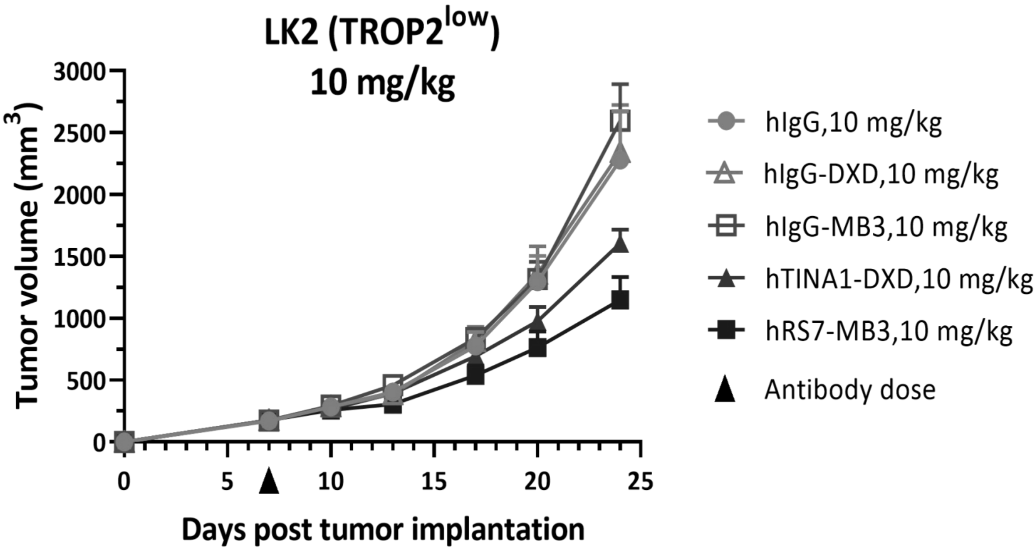


Figure 7

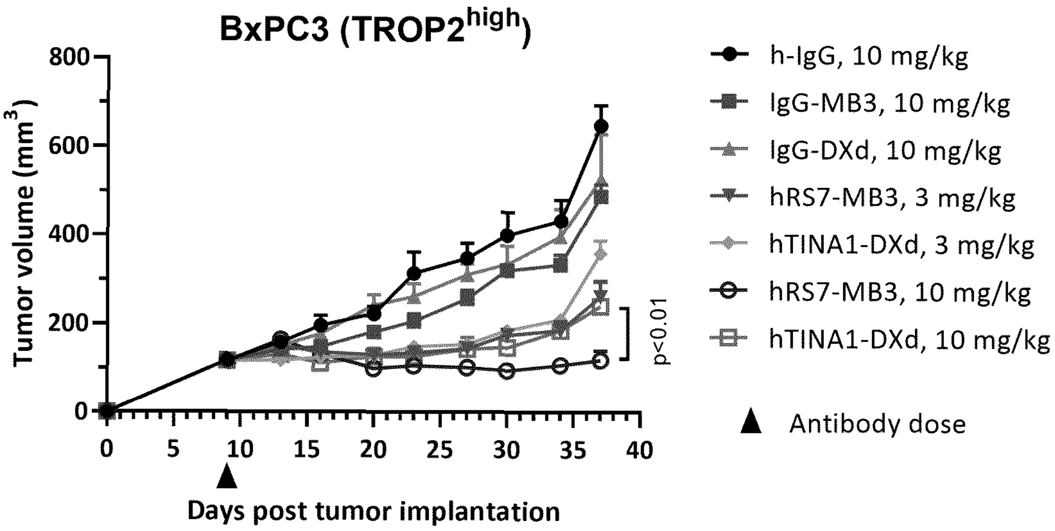


Figure 8

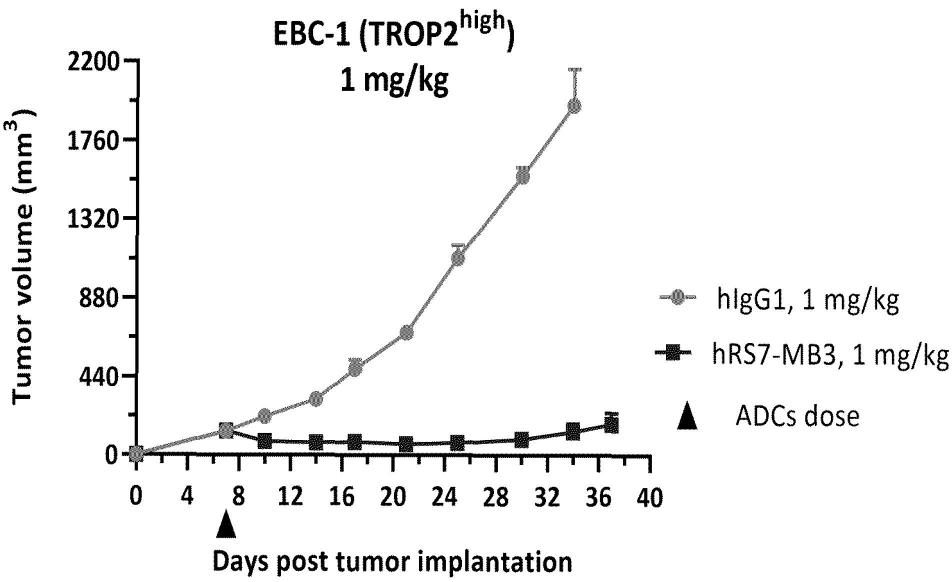


Figure 9

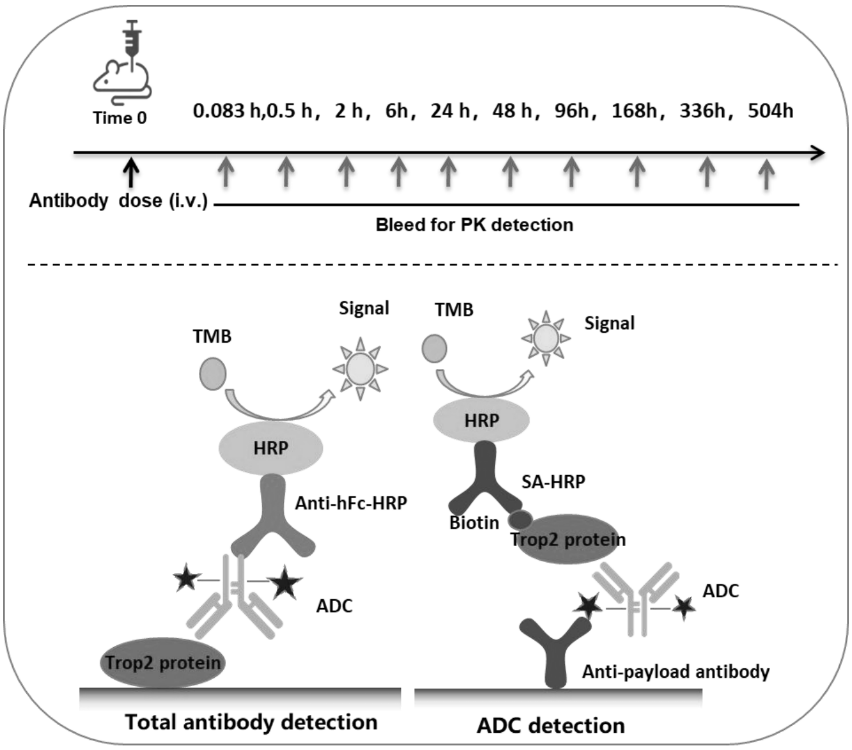


Figure 10A

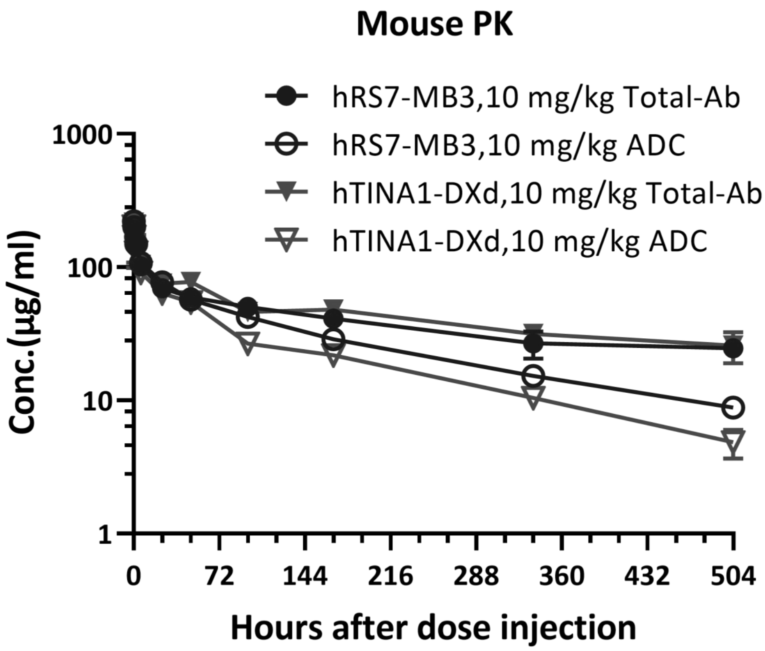


Figure 10B

## INTERNATIONAL SEARCH REPORT

International application No.
**PCT/CN2024/121813**

**A. CLASSIFICATION OF SUBJECT MATTER**

A61K47/68(2017.01)i; C07K16/30(2006.01)i; C07K16/28(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: A61K, C07K, A61P

Documentation searched other than minimum documentation to the extent that such documents are included in the fields searched

Electronic data base consulted during the international search (name of data base and, where practicable, search terms used)

CNABS, CNTXT, DWPI, VEN, ENTXTC, ENTXT, CNKI, PUBMED, ISI_Web of Science, Science Direct, STNext: 信达生物制药(苏州)有限公司, ADC, 偶联物, 缀合物, conjugat+, 喜树碱, camptothecin, TROP2, 序列检索, sequence search, 化合物检索, compound search

**C. DOCUMENTS CONSIDERED TO BE RELEVANT**

| Category* | Citation of document, with indication, where appropriate, of the relevant passages | Relevant to claim No. |
|---|---|---|
| Y | WO 2023138635 A1 (GAN & LEE PHARMACEUTICALS CO., LTD.) 27 July 2023 (2023-07-27)<br>abstract, claims 1-26, and amino acid sequence listing | 1-32 |
| Y | WO 2021173773 A1 (MEDIBOSTON INC.) 02 September 2021 (2021-09-02)<br>abstract, and claims 72-132 | 1-32 |
| Y | WO 2022180581 A2 (MEDIBOSTON LTD.) 01 September 2022 (2022-09-01)<br>abstract, and claims 1-51 | 1-32 |
| Y | WO 2019114666 A1 (SICHUAN KELUN-BIOTECH BIOPHARMACEUTICAL CO., LTD.) 20 June 2019 (2019-06-20)<br>abstract, claims 1-91, and amino acid sequence listing | 1-32 |
| A | CN 109078181 A (BIO-THERA SOLUTIONS, LTD.) 25 December 2018 (2018-12-25)<br>abstract, and claims 1-16 | 1-32 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 January 2025** | **14 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/121813**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/121813**

| Patent document cited in search report | Publication date (day/month/year) | Patent family member(s) | Publication date (day/month/year) |
|---|---|---|---|
| WO 2023138635 A1 | 27 July 2023 | None | |
| WO 2021173773 A1 | 02 September 2021 | JP 2023520605 A | 17 May 2023 |
| | | TW 202146055 A | 16 December 2021 |
| | | US 2021283125 A1 | 16 September 2021 |
| | | US 12029736 B2 | 09 July 2024 |
| | | AU 2021226341 A1 | 29 September 2022 |
| | | CA 3168882 A1 | 02 September 2021 |
| | | MX 2022010457 A | 16 November 2022 |
| | | EP 4110402 A1 | 04 January 2023 |
| | | KR 20230004453 A | 06 January 2023 |
| | | BR 112022017064 A2 | 16 November 2022 |
| WO 2022180581 A2 | 01 September 2022 | JP 2024509099 A | 29 February 2024 |
| | | US 2022378929 A1 | 01 December 2022 |
| | | CA 3208591 A1 | 01 September 2022 |
| | | WO 2022180581 A3 | 13 October 2022 |
| | | KR 20230154892 A | 09 November 2023 |
| | | TW 202302643 A | 16 January 2023 |
| | | EP 4297797 A2 | 03 January 2024 |
| | | AU 2022228004 A1 | 28 September 2023 |
| | | AU 2022228004 A9 | 25 January 2024 |
| WO 2019114666 A1 | 20 June 2019 | EP 3725798 A1 | 21 October 2020 |
| | | EP 3725798 A4 | 10 November 2021 |
| | | US 2020347075 A1 | 05 November 2020 |
| | | US 2021101906 A2 | 08 April 2021 |
| | | US 11970506 B2 | 30 April 2024 |
| | | JP 2024038168 A | 19 March 2024 |
| | | CA 3080236 A1 | 20 June 2019 |
| | | KR 20200099123 A | 21 August 2020 |
| | | US 2023357259 A1 | 09 November 2023 |
| | | JP 2021506743 A | 22 February 2021 |
| | | JP 7446993 B2 | 11 March 2024 |
| | | US 2024382612 A1 | 21 November 2024 |
| CN 109078181 A | 25 December 2018 | CA 3038423 A1 | 14 February 2019 |
| | | JP 2020503243 A | 30 January 2020 |
| | | AU 2018267660 A1 | 28 February 2019 |
| | | AU 2018267660 B2 | 07 May 2020 |
| | | AU 2018267660 C1 | 12 November 2020 |
| | | EP 3464381 A1 | 10 April 2019 |
| | | EP 3464381 A4 | 11 December 2019 |
| | | US 2019048095 A1 | 14 February 2019 |
| | | US 11192954 B2 | 07 December 2021 |
| | | WO 2019029715 A1 | 14 February 2019 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021173773 A
- WO 2015098099 A1
- DD 110102
- WO 2020240467 A1


### Non-patent literature cited in the description


- **YEZHE CHENG et al.** *Frontiers in Oncology*, 23 December 2022, vol. 12, 951589
- **DAISUKE OKAJIMA et al.** *Mol Cancer Ther.*, December 2021, vol. 20 (12), 2329-2340
- **DAVID M GOLDENBERG et al.** *Oncotarget.*, 08 September 2015, vol. 6 (26), 22496-512
- **STEIN et al.** *Antibody Immunocon J. Radiopharm.*, 1991, vol. 4, 703
- **SINGLETON et al.** Dictionary of Microbiology and Molecular Biology. 1994
- Cambridge Dictionary of Science and Technology. 1988
- Glossary of Genetics. Springer Verlag, 1991
- **HALE** ; **MARHAM**. *The Harper Collins Dictionary of Biology*, 1991
- Immunology-A Synthesis. Sinauer Associates, 1991
- *WHO Drug Information*, 2020, vol. 34 (3)
- *Bioconjugate Chem.*, 2015, vol. 26, 919-931